(19) Europäisches Patentamt
European Patent Office
Office européen des brevets

(11) EP 2 487 157 A1

(12) EUROPEAN PATENT APPLICATION

(43) Date of publication:
15.08.2012 Bulletin 2012/33

(51) Int Cl.:
*C07D 215/14* (2006.01) *A61K 31/47* (2006.01)
*A61P 33/06* (2006.01) *A61P 31/04* (2006.01)

(21) Application number: 11154229.6

(22) Date of filing: 11.02.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Université de Picardie Jules Verne
80025 Amiens Cedex 1 (FR)

(72) Inventors:
- Jonet, Alexia
  80025, Amiens Cedex 1 (FR)
- Dassonville-Klimpt, Alexandra
  80025, Amiens Cedex 1 (FR)
- Mullie, Catherine
  80025, Amiens Cedex 1 (FR)
- Taudon, Nicolas
  13262, Marseille Cedex 07 (FR)
- Sonnet, Pascal
  80025, Amiens Cedex 1 (FR)

(74) Representative: Bertrand, Didier
S.A. Fedit-Loriot
38 Avenue Hoche
75008 Paris (FR)

# (54) Enantioselective synthesis method of 4-aminoalcoholquinoline derivatives and the use

(57) Mefloquine derivatives, contrary to chloroquine derivatives have not been widely studied to date. Consequently, mefloquine and its derivatives still remain very attractive synthetic targets. Although mefloquine is usually used clinically as a racemic mixture, some studies have shown that its (+)-enantiomer is more potent than the (-)-enantiomer. Moreover, the (-)-enantiomer is responsible for side effects due to reaction with the central nervous system adenosine receptors, while the (+)-enantiomer does no bind at this binding site. Recently, different libraries of racemate 4-aminoalcoholquinolines showed interesting anti-malarial activities. Herein, the present invention describes an enantiopure synthetic and straightforward route to prepare pure enantiomer 4-aminoalcoholquinoline derivatives through the 4-oxirane key-intermediate. A regioselective $S_N2$ ring opening of this epoxide, by diverse amines, allows to obtain the corresponding (R) or (S) 4-aminoquinolines with good yields and enantiomeric excesses generally superior to 92%. The reported methodology appears suitable for the synthesis of a large number of pure enantiomer 4-aminoalcoholquinoline derivatives.

EP 2 487 157 A1

## Description

The present invention concerns a synthesis method of 4-aminoalcohol quinoline derivatives as well as the compound obtained by the method and the uses of such compounds. Parenthetical document references are listed on the end of the description.

Human malaria is one of the most important diseases in the world, with a corresponding mortality of more than 1 million deaths per year[1]. Four species of *Plasmodium* are responsible for malaria in human beings and among these *P*. *falciparum* is the most dangerous.

Antifolates (pyrimethamine, trimethoprim, sulphonamides) and quinolin-containing drugs (quinine, mefloquine, halofantrine, chloroquine and primaquine) are two principal classes of antimalarial drugs that have been developed during the past 50 years[2]. Newer antimalarial agents such as antibiotics (doxycycline), the hydroxynaphtoquinone derivatives (atovaquone, lumefantrine) and artemisinin (active principle of *Artemesia annua*) were introduced during this period[3]. Due to the efficiency decrease of classical medication towards the rapid extension of *Plasmodium falciparum* chloroquine-resistant strains, there is a need to develop new and effective antimalarial drugs [4]. Extensive work has been done to synthesize chloroquine analogs but much less in regard to mefloquine derivatives.

Consequently, mefloquine and its derivatives still remain very attractive synthetic targets. Mefloquine hydrochloride (Lariam®) is a highly active blood schizontocide against multi-drug resistant *falciparum* malaria strains. This quinoline methanol derivative presents two asymmetric carbon atoms. Karle *et al,* showed that the (+)-enantiomer of the mefloquine is more potent than the (-)-enantiomer by a factor of 1.69 ($IC_{50}$ values against Sierra Leone and Indochina *P*. *falciparum* strains)[5].

However, mefloquine is commonly used clinically as a racemic mixture of (±)-(*R**,*S**)-α-2-piperidinyl-2,8-bis (trifluoromethyl)-4-quinolinemethanol which consists of individual enantiomers (+)-(11*R*,12*S*)-a-2-piperidinyl-2,8-bis(trifluoromethyl)-4-quinolinemethanol and (-)-(11*S*,12*R*)-α-2-piperidinyl-2,8-bis(trifluoromethyl)-4-quinolinemethanol. Mefloquine remains the drug of choice for U.S. military deployments in such regions, primarily because its longer half-life (compared to those of Malarone or doxycycline) [6] allows weekly administration, thereby making compliance less problematic.

However, undesirable side effects on the central nervous system (CNS) have been associated with mefloquine use. These include disturbed sleep, heightened anxiety, panic attacks, depression and psychosis [7,8].

It has been found that the (-)-enantiomer of mefloquine binds to CNS adenosine receptors, while the (+)-enantiomer is without significant activity at this binding site. Moreover, blocking of central adenosine receptors by the (-)-enantiomer is believed to result in neuropsychiatric symptoms associated with mefloquine [9]. Further evidence suggests that the neurotoxicity of mefloquine appears to be related to with the piperidine ring [10]. Previously, it was reported that the opening of the piperidine ring at the 4-position of the quinoline scaffold is associated with an improved potency and a selectivity relative to mefloquine.

Recently, Dow *et al*. described the antimalarial potential together with the physicochemical properties of several libraries describing racemic mixtures of mefloquine non-piperidine analogs [11]. According to these authors, the mefloquine neurological effects could be limited by a lower permeability of the blood-brain barrier of new mefloquine analogs through physicochemical properties modifications. From their library, an active series of diamines has been identified showing a similar metabolic stability and a lower permeability than mefloquine. The main modification of the mefloquine derivatives concerns the 4-position of the quinoline scaffold to synthesize 4-aminoalcoholquinoline [11,12]. Racemic mixtures of mefloquine analogs were synthesized and evaluated for other biological properties such as treatment of tuberculosis [13], bacterial infections and, treatment of movement disorders (Parkinson's or Alzheimer's diseases) due to their binding affinity to adenosine $A_2$ receptor [14].

Several other approaches to reduce mefloquine neurotoxicity have been proposed: (i) reformulation of mefloquine as a pure isomer, and (ii) reengineering of mefloquine (based on physicochemical properties modifications) to obtain less neurotoxic derivatives still showing an antimalarial activity. In the continuation of the inventor's work concerning the synthesis of new antimalarial drugs, the asymmetric synthesis of enantiopure 4-aminoalcoholquinolines mefloquine analogs have been considered as an interesting goal. These compounds should present the physiochemical properties required to achieve a balance between potency, reduced blood-brain barrier penetration, and metabolic stability, However, until now, no enantioselective syntheses of mefloquine amino-analogs have been described,

Consequently, the purpose of the present invention is to propose a new enantioselective pathway to mefloquine amino-analogs allowing the access of new antimalarial compounds with a strong antimalarial activity as well as antibacterial activity and few neurological side effects.

To this end, there is provided a process for providing a compound of formula (I):

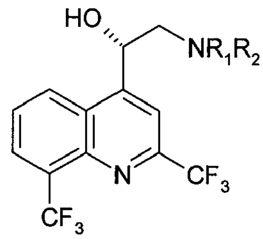

(I)

Wherein:

- $R_1$ and $R_2$ are selected from a group consisting of a hydrogen atom, a chain alkyl group ($C_1$-$C_9$), a cyclic alkyl groups and aromatic groups; or
- $NR_1R_2$ forms together a piperazine or a piperidine substituted by at least one of a hydrogen, a chain alkyl group ($C_1$-$C_9$), a cyclic alkyl group, a chain containing an aromatic group and a chain containing a heterocyclic group, the process comprising steps of in the following order: preparation of 2,8-bis(trifluoromethyl)-4-vinylquinoline;
- preparation of *(S)*-1-[2,8-bis(trifluoromethyl)quinolin-4-yl]ethane-1,2-diol by adding an asymmetric dihydroxylation catalyst to the 2,8-bis(trifluoromethyl)-4-vinylquinoline;
- preparation of *(S)*-4-(oxiran-2-yl)-2,8-bis(trifluoromethyl) quinoline starting from the *(S)*-1-(2,8-bis(trifluoromethyl) quinolin-4-yl]ethane-1,2-diol; and
- adding an amine R1R2 to the *(S)*-4-(oxiran-2-yl)-2,8-bis(trifluoromethyl)quinoline.

The invention further relates to a process for providing a compound of formula (II):

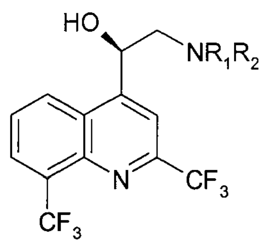

(II)

Wherein:

- $R_1$ and $R_2$ are selected from a group consisting of a hydrogen atom, a chain alkyl group ($C_1$-$C_9$), a cyclic alkyl groups and aromatic groups; or
- $NR_1R_2$ forms together a piperazine or a piperidine substituted by at least one of a hydrogen, a chain alkyl group ($C_1$-$C_9$), a cyclic alkyl group, a chain containing an aromatic group and a chain containing a heterocyclic group, the process comprising steps of in the following order:
- preparation of 2,8-bis(trifluoromethyl)-4-vinylquinoline;
- preparation of *(R)*-1-[2,8-bis(trifluoromethyl)quinolin-4-yl]ethane-1,2-diol by adding an asymmetric dihydroxylation catalyst to the 2,8-bis(trifluoromethyl)-4-vinylquinoline;
- preparation of *(R)*-4-(oxiran-2-yl)-2,8-bis(trifluoromethyl) quinoline starting from the *(R)*-1-[2,8-bis(trifluoromethyl) quinolin-4-yl]ethane-1,2-diol; and
- adding an amine R1R2NH to the *(R)*-4-(oxiran-2-yl)-2,8-bis(trifluoromethyl)quinoline.

The present invention enables to provide an enantiopure, synthetic, and straightforward route to prepare 4-aminoquinolines derivatives through the enantiopur 4-oxirane synthesis. The preparation of *(S)*-or *(R)*- 4-(oxiran-2-yl)-2,8-bis(trifluoromethyl) quinoline is carried out from 4-vinylquinoline in two steps *via* a Sharpless asymmetric dihydroxylation with retention of configuration. The key-intermediate enantiopure 4-(oxiran-2-yl)-2,8-bis(trifluoromethyl) quinoline has been easily diversified, on position 4, through a regioselective $S_N2$ ring opening mechanism with various amines, to provide the corresponding enantiopure 4-aminoquinolines (*R*) or (*S*) with good yield. In consequent, the fabrication cost of the antimalarial and antibacterial drugs can be drastically reduced.

Preferably, the 2,8-bis(trifluoromethyl)-4-vinylquinoline is prepared by reacting a 4-bromo-2,8-bis(trifluoromethyl)quinoline with vinylstannane using the $Pd(PPh_3)_2Cl_2$ as catalyst and tetrabutylammonium bromide as additive in

reflux of dried acetonitrile. According to this feature, the yield and purity of 2,8-bis(trifluoromethyl)-4-vinylquinoline can be improved.

Alternatively, the 2,8-bis(trifluoromethyl)-4-vinylquinoline is prepared by reacting 4-bromo-2,8-bis(trifluoromethyl)quinoline with dibutyl vinylboronate.

Alternatively, the 2,8-bis(trifluoromethyl)-4-vinylquinoline is prepared by reacting 4-bromo-2,8-bis(trifluoromethyl)quinoline with dibutyl vinylboronate.

Suitably, the asymmetric dihydroxylation catalysts is one of AD-mix $\alpha$ and AD-mix $\beta$, and the *(S)* -1-[2,8-bis (trifluoromethyl)quinolin-4-yl]ethane-1,2-diol is prepared by reacting said 2,8-bis(trifluoromethyl)-4-vinylquinoline with AD-mix $\alpha$ in a solution of t-BuOH/$H_2O$ at 0˚C, and the *(R)* - 1-[2,8-bis(trifluoromethyl)qinolin-4-yl]ethane-1,2-diol is prepared by reacting said 2,8-bis(trifluoromethyl)-4-vinylquinoline with AD-mix $\beta$ in a solution of t-BuOH/$H_2O$ at 0˚C. $K_2OsO_2(OH)_4$ may be further added to prepare *(S)*-1-[2,8-bis(trifluoromethyl)quinolin-4-yl]ethane-1,2-diol and *(R)* - 1-[2,8-bis(trifluoromethyl)quinolin-4-yl]ethane-1,2-diol. Since both AD-mix $\alpha$ and AD-mix $\beta$ are commercially available, the process can be easily realised.

Preferably yet, starting from *(S)*-1-[2,8-bis(trifluoromethyl)quinolin-4-yl]ethane-1,2-diol (4b) and, *(S)*-4-(oxiran-2-yl)-2,8-bis(trifluoromethyl) quinoline (2b) is prepared by a process comprising steps of:

- formation of the cyclic orthoester by acid catalyzed transesterification;
- generation of halohydrins ester via regioselective opening of acetoxonium ion by addition of tributyldimethylsilyl chloride; and
- cyclization to epoxide by base mediated saponification in methanol. This process may also be used to prepare *(R)*-4-(oxiran-2-yl)-2,8-bis(trifluoromethyl) quinoline (2a) starting from *(R)*-1-[2,8-bis(trifluoromethyl)quinolin-4-yl] ethane-1,2-diol (4a).

The invention further relates to the compound of compound of formula (I) or (II):

(I) 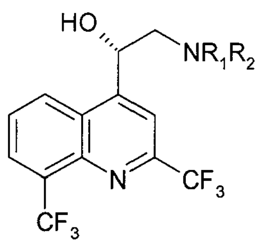

(II) 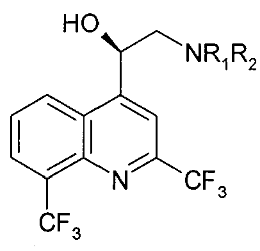

Wherein:

- $R_1$ and $R_2$ are selected from a group consisting of a hydrogen atom, a chain alkyl group ($C_1$-$C_9$), a cyclic alkyl groups and aromatic groups; or
- NR1R2 forms together a piperazine or a piperidine substituted by at least one of a hydrogen, a chain alkyl group ($C_1$-$C_9$), a cyclic alkyl group, a chain containing an aromatic group and a chain containing a heterocyclic group. The advantage of the present invention lie in that, new antimalarial agents and antibacterial agents are obtained by providing enantiopure 4-aminoquinolines.

Preferably, the formula (I) is one of *(S)*-2-(benzylamino)-1-(2,8-bis (trifluoromethyl)quinolin-4-yl)ethanol, *(S)*-ethyl-4-(4-(2-(2,8-bis(trifluoromethyl)quinolin-4-yl)-2-(hydroxyethyl)piperazin-1-yl)butanoate, *(S)*-1-(2,8-bis(trifluoromethyl)quinolin-4-yl)-2-(pentylamino)ethanol, *(S)*-1-(2,8-bis(trifluoromethyl)quinolin-4-yl)-2-(hexylamino) ethanol, *(S)*-1-(2,8-bis(trifluoromethyl)quinolin-4-yl)-2-(heptylamino)ethanol,*(S)*-1-(2,8-bis(trifluoromethyl)quinolin-4-yl)-2-(octylamino)ethanol, *(S)*-1-(2,8-bis(trifluoromethyl) quinolin-4-yl)-2-(nonylamino)ethanol *(S)*-1-(2,8-bis(trifluoromethyl)quinolin-4-yl)-2-(piperazine substituted)ethanol or *(S)*-tert-butyl-(3-(4-(3((2-(2,8-bis(trifluoromethyl) quinolin-4-yl)-2-(hydroxyethyl) amino)propyl)piperazin-1-yl)propyl)carbamate.

Alternatively, the formula (II) is one of *(R)*-2-(benzylamino)-1-(2,8-bis (trifluoromethyl)quinolin-4-yl)ethanol, *(R)*-ethyl-4-(4-(2-(2,8-bis(trifluoromethyl)quinolin-4-yl)-2-(hydroxyethyl)piperazin-1-yl)butanoate, *(R)*-1-(2,8-bis(trifluoromethyl)quinolin-4-yl)-2-(pentylamino)ethanol, *(R)*-1-(2,8-bis(trifluoromethyl)quinolin-4-yl)-2-(hexylamino) ethanol, *(R)*-1-(2,8-bis(trifluoromethyl)quinolin-4-yl)-2-(heptylamino)ethanol,*(R)*-1-(2,8-bis(trifluoromethyl)quinolin-4-yl)-2-(octylamino)ethanol, *(R)*-1-(2,8-bis(trifluoromethyl) quinolin-4-yl)-2-(nonylamino)ethanol *(R)*-1-(2,8-bis(trifluoromethyl)quinolin-4-yl)-2-(piperazine substituted) ethanol or *(R)*-tert-butyl-(3-(4-(3((2-(2,8-bis(trifluoromethyl) quinolin-4-yl)-2-(hydroxyethyl) amino)propyl)piperazin-1-yl)propyl)carbamate.

The invention further relates to the use of the compound (I) or (II) as an antimalarial agent.

Alternatively, the invention relates also to the use of the compound (I) or (II) as an antibacterial agent.

The present invention is also intended to provide a pharmaceutical composition comprising the compound of the formula (I) or (II). According to the present invention, compounds with excellent antimalarial activity and antibacterial activity can be obtained.

The present invention will be described in further detail with reference to the accompanying drawings wherein:

Fig. 1 shows Mefloquine Enantiomer Structures;
Fig. 2 shows a scheme of Retrosynthesis of (11*R*)- Enantiopure 4-Aminoalcoholquinoline 1;
Fig. 3 shows a scheme of synthesis of 2, 8-trifluoromethyl-4-vinylquinoline 3.
Fig. 4 shows a scheme of Suzuki Cross-Coupling;
Fig. 5 shows a scheme of direct oxirane synthesis using the Chiral (salen)Mn(III) Complexes;
Fig. 6 shows preferred Conformations of MTPA Esters;
Fig.7 shows a scheme of reparation of Mosher's monoester 8a and 8b;
Fig. 8 shows $^1$H NMR Analysis of the Diastereomeric Esters 8a and 8b;
Fig. 9 shows a scheme of one-pot Stereospecific Conversion of the 1,2-diol 4a into Epoxyde 2a *via* a Chlorhydrine ester; and
Fig. 10 shows a View of the Crystal Structure of (*R*)-2a with our Numbering Scheme, Displacement Ellipsoids Are Drawn at the 30% Probability Level.

Mefloquine and its synthesis were first described by Ohnmacht *et al.* [15] in 1971. In 1974, a more detailed account of the synthesis along with anti-malarial activity of the mefloquine isomers is given by Carroll and Blackwell [16]. More recently, Xie *et al.* reported a new and enantioselective synthesis of the (11*R*,12*S*)-mefloquine hydrochloride using a proline-catalyzed asymmetric direct aldol reaction and a Beckmann rearrangement as key steps. The experimental analysis confirmed the absolute configuration of (+)-mefloquine as (11*R*,12*S*) [17] (see Figure 1).

During the synthesis of *R*- and *S*-4aminoalcoholquinoline mefloquine analogs 1, the envisaged strategy involved enantiopure key intermediate 2 (4-oxirane) synthesis, (Figure 2). This enantioselective epoxide can be easily diversified on position 4, through a regioselective $S_N2$ ring opening mechanism. The preparation of 4-oxirane 2 was carried out from 4-vinylquinoline 3 either in one step by a Jacobsen epoxidation or in two steps *via* a Sharpless asymmetric dihydroxylation (diol 4).

The 4-vinylquinoline 3 has been prepared from the corresponding 4-bromoquinoline or 4-sulfoxyquinoline 6 (4-tosyloxy- or 4-trifluorooxy-quinoline) (figure 3). The 4-bromoquinoline 6a is easily accessible by reaction of 4-hydroxyquinoline 5 with phosphorus oxybromide at 150 °C. Sulfonates 6b and 6c are prepared from the same alcohol 5 by action of triflic anhydride (50% yield) and p-toluenesulfonic acid (96% yield), respectively. These different precursors will be used as electrophile in transition-metal-catalyzed cross couplings (Hiyama, Stille and Suzuki reactions).

Using the reaction conditions reported by literature, [18] a Hiyama coupling has been performed with vinyltrimethoxysilane and 2,8-trifluoromethyl-4-bromoquinoline 6a on water using sodium hydroxide as the activator at 120 °C. Normally, this process should give good results in presence of low quantity of diacetate palladium (1 mol %). Unfortunately, in our case, 4-vinylquinoline 3 is formed in only 14% yield after 16 h of reaction.

To further reduce time reaction and to increase the yield, the Stille coupling reaction with vinyltributylstannane as a vinylating reagent has been investigated. In order to determine the optimal cross-coupling reaction conditions (solvents, catalysts, substrates and bases) a study has been carried out. The results are shown in Table 1. A first assay was performed by using the reaction conditions described by Comins *et al.* in 2005, slightly modified [19].

The 4-bromoquinoline 6a was reacted with vinylstannane using the $Pd(PPh_3)_2Cl_2$ as catalyst and tetrabutylammonium bromide as additive in reflux of dried acetonitrile (entry 1, table 1). The 4-vinyl product has been obtained in 46% yield with a purity of 98% determined by HPLC. The starting material was totally converted but the reaction suffered of instability of the catalyst, which was observable through the precipitation of black palladium over the course of the reaction. Thus, when the same reaction was performed using oven dried glassware and distilled acetonitrile: a yield increase of 20% was observed (entry 2). The comparison between three solvents (dimethylformamide, toluene and acetonitrile) revealed that acetonitrile affords the best yields (entries 2-5). Without adding tetrabutylammonium bromide (TBAB), a 43% yield (vs. 65%) of the corresponding cross-coupled product 3 was obtained (entry 5). Indeed, TBAB is known to be able to stabilize colloidal palladium nanoparticules that act as catalysts.

**Table 1**. Stille Coupling Conditions to Prepare Alkene 3.

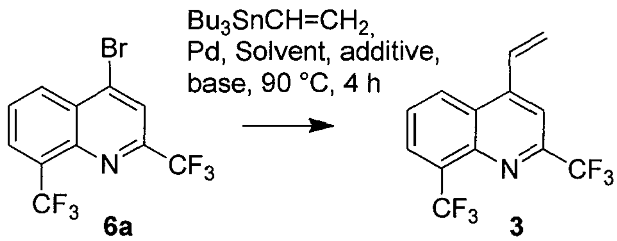


6a 3

| Entry[a] | Pd catalyst (mol %) | TBAB (eq.) | Base (eq.) | Solvent[b] | Yield (%)[d] | Purity (%)[e] |
|---|---|---|---|---|---|---|
| 1 | $Pd(PPh_3)_2Cl_2$ (10) | 1 | - | MeCNc | 46 | 98 |
| 2 | $Pd(PPh_3)_2Cl_2$ (10) | 1 | - | MeCN | 65 | nd |
| 3 | $Pd(PPh_3)_2Cl_2$ (10) | 1 | - | Toluene | 7 | nd |
| 4 | $Pd(PPh_3)_2Cl_2$ (10) | 1 | - | DMF | 13 | nd |
| 5 | $Pd(PPh_3)_2Cl_2$ (10) | - | - | MeCN | 43 | 99 |
| 6 | $Pd(PPh_3)_4$(10) | 1 | 2 | MeCN | 9 | nd |
| 7 | $Pd(PPhs)_2Cl_2$ (10) + $PPh_3$ (20) | 1 | - | MeCN | 16 | 96 |
| 8 | $Pd_2(dba)_3$ (10) + $PPh_3$ (20) | 1 | - | MeCN | 84 | 72 |
| 9 | $Pd(OAc)_2$(10) | 1 | - | MeCN | 60 | 83 |
| 10 | $Pd(PPh_3)_2Cl_2$ (10) | 1 | 2 | MeCN | 75 | 100 |
| 11 | $Pd_2(dba)_3$(10) | 1 | 2 | MeCN | 88 | 68 |
| 12 | $Pd(PPh_3)_2Cl_2$ (5) | 1 | 2 | MeCN | 48 | 96 |

[a] All reactions were performed with 1 eq. of 6a and 2 eq. of $BusSnCH=CH_2$.
[b] Performed using dry solvents.
[c] Performed using a degassed solvent.
[d] yield obtained after flash chromatography.
[e] Determined by HPLC analysis.

Other Pd complexes tested as catalysts have been less effective than $Pd(PPh_3)_2Cl_2$ (entries 8 and 9 of table 1). The addition of an inorganic base like $K_2CO_3$ increases reaction yield (entries 10 *vs*. 2). The best yield with a satisfactory purity is obtained using $Pd(PPh_3)_2Cl_2$, TBAB, distilled acetonitrile and $K_2CO_3$ (75%, entry 10). When 4-bromoquinoline was used in the place of 4-tosyloxy- and 4-trifluorooxyquinoline in the optimal conditions previously described, the yields increased in an important way by 42 and 60%, respectively.

The vinylquinoline 3 easily prepared in good yield (82%) by a direct Suzuki-Miyaura cross-coupling reaction of 4-bromoquinoline 6a with dibutyl vinylboronate (figure 4). This step has been performed in the presence of $Pd(PPh_3)_4$ as a catalyst and a 4 M aqueous solution of KOH solution [20]. The Suzuki-Miyaura-type reaction was expanded with potassium vinyltrifluoroborate and 4-bromoquinoline 6a as coupling partner by using $PdCl_2(dppf)\cdot CH_2Cl_2$ as the catalyst, $Cs_2CO_3$ as the base, and THF-$H_2O$ as the solvent system [21]. Consequently, this new palladium-catalyzed cross-coupling reaction led to the expected 4-vinylquinoline 3 with a 71 % yield (see figure 5).

After optimization of the 4-vinylquinoline 3 preparation, a two step process synthesis based on an asymmetric dihydroxylation chemistry followed by a stereospecific cyclization is realized. Alternatively a direct asymmetric epoxidation using a chiral (salen)Mn catalyst may be used.

The cyclization can be obtained according to different literature procedures: (i) a base-mediated ring closure *via* selective hydroxyl activation, generally in a mesylate or tosylate form [22], (ii) a formation of acetoxy halides and epoxide *via* the Sharpless acetoxonium ion[23] or (iii) a Mistunobu reaction [24]. The Sharpless asymmetric dihydroxylation method followed by a cyclization via Sharpless acetoxonium ion is chosen in the present invention. The inventors have developed the optimized conditions of the Sharpless asymmetric dihydroxylation starting from the 2,8-bis(trifluoromethyl)-4-vinylquinoline (3).

According to the final enantiomeric alcohol expected 4a (*R*) or 4b (*S*) the vinylquinoline 3 is put to react with commercially available AD-mix $\alpha$ or AD-mix $\beta$, in a solution of *t*-BuOH/$H_2O$ at 0˚C, with eventual addition of $K_2OsO_2(OH)_4$ and $MeSO_2NH_2$ (see table 2). Alternatively, divers asymmetric dihydroxylation catalysts may be used in the place of the AD-mix as far as it fulfills the same role as the AD-mix.

**Table 2**. Reagents and Conditions Used to Prepare Enantiopure Diol **4a** and **4b**.

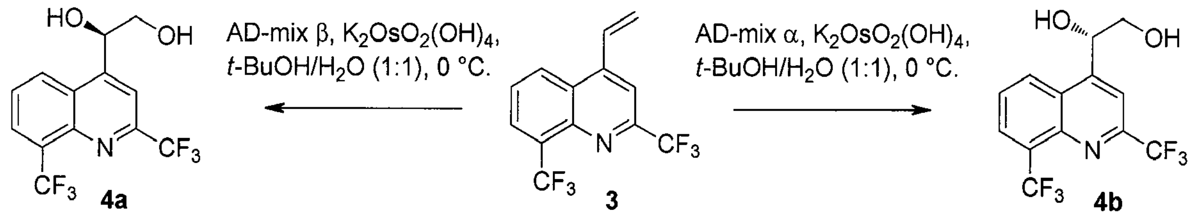


**4a** **3** **4b**

| Entry | AD-mix (g) | $K_2OsO_2(OH)_4$ | $MeSO_2NH_2$ | Time (h) | Yield (%) | % ee[c] | $[\alpha]_D^{20}$ [e] | Diol (Absolute configuration)[f] |
|---|---|---|---|---|---|---|---|---|
| 1 | α (1.4)[a] | - | 1 mmol | 26 | 37 | nd[d] | - | - |
| 2 | β (1.4)[b] | - | 1 mmol | 30 | 43 | nd | - | - |
| 3 | α (2.5) | - | 1 mmol | 28 | 36 | nd | - | - |
| 4 | α (6.4) | - | 1 mmol | 47 | 26 | 96 | + 62 | **4b** (*S*) |
| 5 | β (1.4) | 1 mol % | 1 mmol | 24 | 78 | 98 | - 52 | **4a** (*R*) |
| 6 | β (1.4) | 1 mol % | - | 19 | 68 | 97 | - 52 | **4a** (*R*) |
| 7 | α (1.4) | 1 mol % | - | 18 | 78 | 96 | + 62 | **4b** (*S*) |

[a] 1 g of AD-mix a corresponds to 1.0 mol % of $(DHQ)_2$-PHAL, 3 mmol of $K_3Fe(CN)_6$, 3 mmol of $K_2CO_3$, 0.4 mol % of $K_2OsO_2(OH)_4$.
[b] 1 g of AD-mix β corresponds to 1.0 mol % of $(DHQD)_2$-PHAL, 3 mmol of $K_3Fe(CN)_6$, 3 mmol of $K_2CO_3$, 0.4 mol % of $K_2OsO_2(OH)_4$.
[c] Enantiomeric excesses were determined by HPLC (Chiralpak IB column, heptane/*i*-PrOH 90:10; 1mL/min, 210 nm) $t_r$ (*R*) = 26 min, $t_r$ (*S*) = 33 min;
[d] not determined.
[e] *c* 0.25, DCM.
[f] Configuration of the major enantiomer was determined by analyzing $^1H$ NMR spectra of the Mosher monoesters and/or with crystallographic epoxide spectra.

The first assays of asymmetric dihydroxylation were carried out with 1.4 g of AD-mix α or β added to 1 equivalent of methylsulfonamide (entries 1, 2) as described by Kolb *et al.* [25] The corresponding oxiranes 4a or 4b were obtained with 40% yield average. In order to increase the yield, quantities of AD-mix α have been increased: doubled (entry 3) or multiplied by four (entry 4). Addition of 1 mol % of $K_2OsO_2(OH)_4$ to 1.4 g of AD-mix increased significantly the yield (78% vs. 43% entries 5, 2).

Furthermore, the inventors demonstrated that the presence of methylsulfonamide is not required for the advancement of the reaction (entry 5, 6). Finally, the treatment of vinylquinoline 3 with 1.4 g of AD-mix β in the presence of 1 mol % of oxidant $K_2OsO_2(OH)_4$ provided the corresponding (*R*)-diol ([α]$D^{20}$ -52 (c 0.25; DCM)) in 68% yield and 97% enantiomeric excess (ee). The (*S*)-diol ($[\alpha]_D^{20}$ +62 (c 0.25; DCM)) was prepared from 1.4 g of AD-mix α in the presence of 1 mol % of oxidant $K_2OsO_2(OH)_4$ in 78% yield and 96% ee.

The Mosher's MTPA (methoxy(trifluoromethyl)phenylacetyl) method is a well-known tool to determine the absolute configuration of chiral alcohols and primary amines since this process does not require crystallization of compounds [26], In MTPA esters, the aromatic substituent (phenyl group) generates a diamagnetic anisotropy effect due to the ring current induced by the external magnetic field. The proton NMR signals of the alcohol moiety facing the phenyl group on the preferred conformation are moved to a higher magnetic field (high field shift). In ester (*S*,*X*), the protons of group $R_1$ feel a diamagnetic anisotropy effect, and hence their $^1$H NMR signals show high-field shifts whereas $R_2$ does not (figure 6). Conversely, in the ester (*R*, *X*), $R_2$ is above the benzene plane of the MTPA moiety, and hence the protons of group $R_2$ show high-field shifts. The parameter Δδ reflecting the anisotropy is defined as Δδ = δ(*S*,*X*) - δ(*R*, *X*) (see figure 6).

The corresponding Mosher's monoester 8 was prepared, from the diol 4a (*R*), using (*S*)-α-methoxy-α-trifluoromethyl-phenylacetic acid or (*R*)-α-methoxy-a-trifluoromethyl-phenylacetic acid. To this purpose, the alcohol 7 was obtained with a 39% yield after selective primary alcohol protection of compound 4a with t-butyldimethylsilylchloride in DMF, at room temperature. Subsequently, the two diastereomeric esters 8a (*R,R*) and 8b (*S,R*) were synthesized by reaction of 7 with the corresponding (*R*)- and (*S*)-Mosher's MTPA in dichloromethane with a 49% yield (see figure 7).

$^1$H NMR analyses of the diastereomeric esters allowed us to establish without ambiguity the absolute configuration of the diols by combining the anisotropy effects discussed above with the definition of Δδ parameter. The MTPA moiety and the methine proton of the secondary alcohol moiety are placed in the up in the front side and in the rear side, respectively. The substituent $R_2$ showing positive Δδ values is placed at the right side, while the substituent $R_1$ showing negative Δδ values is at the left side. So, the absolute configuration (*X*=*R*) of our alcohol has been determined (Figure 8). Concerning the epoxide formation by ring closure *via* Sharpless acetoxonium ion, we have chosen to use a "one-pot" method, previously describe by Sharpless for the stereospecific conversion of each *R* or *S* 1,2-diols into their corresponding epoxides[3]. This process involves three successive steps: (i) formation of the cyclic orthoester by acid catalyzed transesterification. (ii) generation of halohydrins ester *via* regioselective opening of acetoxonium ion by addition of tributyldimethylsilyl chloride and (iii) cyclization to epoxide by base mediated saponification in methanol. This conversion of the diol 4a to the oxirane 2a was accomplished with global retention of configuration because this process involves two successive inversions at the same stereocenter: (i) inversion at the halide receiving stereocenter at the time of halohydrins ester formation and (ii) second inversion at the halide center while cyclization to epoxide (see figure 9).

All three operations have been carried out in one reaction vessel without isolation of any intermediates but each step was followed and validated by GCMS. Thus, the slightly modified Sharpless conditions applied to the conversion of diols 4a and 4b give the corresponding oxiranes 2a and 2b with retention of configuration according to optical rotation of each compound (Table 3).

**Table 3**. Substrate Scope of Enantioselective Epoxydation

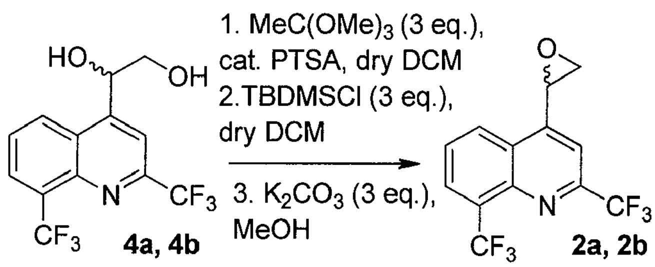


**4a, 4b** **2a, 2b**

| 4 | 2 | Yield (%) | ee (%)[a] | $[\alpha]_D^{20}$ [b] | Absolute configuration |
|---|---|---|---|---|---|
| **a** | **a** | 68 | 92 | -52 | *R* |

(continued)

| 4 | 2 | Yield (%) | ee (%)[a] | $[\alpha]_D^{20}$ [b] | Absolute configuration |
|---|---|---|---|---|---|
| **b** | **b** | 57 | 96 | +45 | *S* |

[a] Enantiomeric excess was determined by GC.
[b] *c* 0.25, MeOH.
[c] Absolute configuration was determined with crystallographic epoxide spectra.

In order to confirm the structure and the absolute configuration of the synthesized quinoline epoxides 2, X-rays studies for the two derivatives were performed. Unfortunately, suitable crystals were only obtained for compound (-)-2a. Its molecular structure, depicted in Figure 10, confirms the structure in the solid state as anticipated on the basis of IR and NMR data. Moreover, the configuration of (-)-2a was determined by observing and calculating the *F*(+)/*F*(-) ratios of Bijvoet pairs using the mean *F* value of each independent reflection.[27] Based on these results, the absolute configuration at C-11 in (-)-2a is determined to be *R*. And consequently (-)-2a led us to consider that the (+)-enantiomer-2b should present the S-absolute configuration. Furthermore, these results indirectly confirm the stereochemistry of each *R* or *S* quinolin-4-yl-ethanediol precursors 4 (see figure 10),

Finally, the treatment of oxirane 2a or 2b with diverse amines, in a regioselective $S_N2$ nucleophilic ring opening mechanism, provided the corresponding enantiopure 4-aminoquinolines 1a-e (*R*) or 1f-j (*S*) with good yield (35-96%) and ees generally superior to 92% (Table 4). No epimerization at the stereogenic carbon was observed, as determined by chiral HPLC analysis, using the opposite enantiomer as a standard.

**Table 4. (11*R*)** pure 4-aminoquinolines derivatives synthesis by regioselective $S_N2$ epoxyde opening.

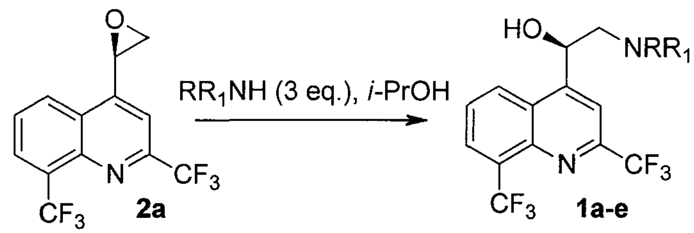


2a → 1a-e

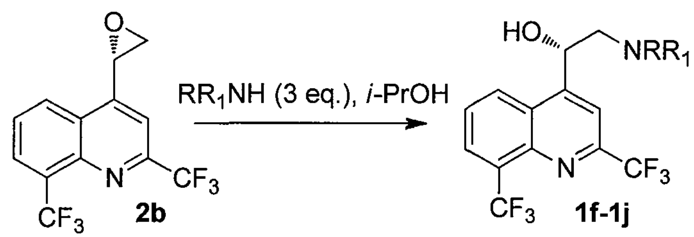


2b → 1f-1j

| Entry | Substrate: $RR_1NH$ | Product | Yield (%) | ee (%)[a] | $[\alpha]_D^T$ | Absolute configuration[b] | $t_r^c$ (min) |
|---|---|---|---|---|---|---|---|
| 1 | 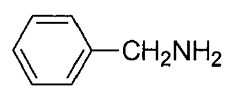 | **1a** | 66 | 95 | -34.5[d] | *R* | 16.8 |
| 2 | | **1f** | 89 | 98 | +46.3[d] | *S* | 18.9 |
| 3 | 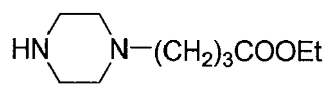 | **1b** | 35 | 89 | -70.7[e] | *R* | 9.0 |
| 4 | | **1g** | 38 | 97 | +78.2[e] | *S* | 20.1 |
| 5 | *n*-$C_5H_{11}NH_2$ | **1c** | 88 | 94 | -50.3[f] | *R* | 21.5 |
| 6 | | **1h** | 93 | 99 | +49.8[f] | *S* | 25.3 |
| 7 | *n*-$C_6H_{13}NH_2$ | **1d** | 88 | 94 | -47.5[f] | *R* | 18.5 |
| 8 | | **1i** | 85 | 98 | +54.0[f] | *S* | 21.4 |

(continued)

| Entry | Substrate: $RR_1NH$ | Product | Yield (%) | ee (%)[a] | $[\alpha]_D^T$ | Absolute configuration[b] | $t_r^c$ (min) |
|---|---|---|---|---|---|---|---|
| 9 | | **1e** | 98 | 93 | -49.6[e] | *R* | 6.2 |
| 10 | 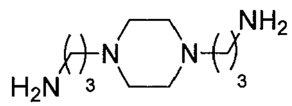 | **1j** | 82 | 92 | +50.6[e] | *S* | 7.2 |

[a] Enantiomeric excess was determined by Chiral HPLC.
[b] Absolute configuration was determined with epoxide crystallographic spectra.
[c] Retention time.
[d] *c* 0.25, DMSO.
[e] *c* 0.25, DCM.
[f] *c* 0.25, MeOH.

Previous work by Milner *et al.* [12] described an exploratory 4-alcoholquinolines library meant to provide early-lead racemate compounds. The present invention is intended to provide an enantiopure, synthetic, and straightforward route to prepare 4-aminoquinolines derivatives through the enantiopur 4-oxirane 2 synthesis. The preparation of 2 was carried out from 4-vinylquinoline 3 in two steps *via* a Sharpless asymmetric dihydroxylation with retention of configuration. The key-intermediate 2 has been easily diversified, on position 4, through a regioselective $S_N2$ ring opening mechanism with various amines, to provide the corresponding enantiopure 4-aminoquinolines 1 a (*R*) or 1b (*S*) with good yield (35-96%) and ees generally superior to 92%. The antimalarial activity of these compounds will be tested *in vitro* and *in vivo.*

**Example 1 General methods**

All starting materials and reagents were obtained from commercial suppliers and were used without further purification. Reactions requiring anhydrous conditions were performed under a blanket of argon. All solvents were purified via literature procedures or used without further purification. Flash column chromatography was carried out on Kielselgel 60 (40-63 μm) ASTM (Merck). Routine monitoring of reactions was performed using Merck silica gel 60 F254 plates, thin layer chromatography (TLC) and visualized under UV light (254 nm), with ethanolic phosphomolybdic acid (PMA). Nuclear magnetic resonance (NMR) spectra were recorded using Bruker 600 MHz NMR instrument ($^1$H NMR at 600 MHz and $^{13}$C NMR at 150 MHz) and Bruker 300 MHz NMR instrument ($^1$H NMR at 300 MHz and $^{13}$C NMR at 75 MHz). Chemical shifts are expressed in parts per million (δ, ppm) downfield from tetramethylsilane and are referenced to the deuterated solvent. $^1$H NMR and $^{13}$C NMR data were reported in the order of chemical shift, multiplicity (s = singlet, d = doublet, t = triplet, q = quartet, qt = quintuplet, m = multiplet), integration, coupling constants in hertz (Hz). High Performance Liquid Chromatography (HPLC) was carried out on a Schimadzu LC-20AD equipped with a Chiralpak IB column. Specific rotations were measured on a Jasco P1010 polarimeter. High-resolution mass spectra were obtained from a Micromass-Waters Q-TOF Ultima spectrometer. Infrared spectra were recorded with a Jasco FTIR-4200 and are reported using the frequency of absorption ($cm^{-1}$). Gas Chromatography-Mass Spectrometry (GCMS) was carried out on a Schimadzu GCMS-QP2010S equipped with a SLB-5ms.

**4-bromo-2,8-bis(trifluoromethyl)quinoline (6a)**

Phosphorous oxybromide (4 g, 14.2 mmol), under argon atmosphere, was heated to 90 ˚C until complete dissolution of the solid. **5** (4.08 g, 14.2 mmol) was added to this hot solution and the bath temperature was increased to 150 ˚C. After 6 h, the resulting mixture was allowed to cool to room temperature. The reaction mixture was quenched by addition of ice-cold water and the precipitated formed was filtered and washed with water to afford the expected compound **6a** (4.70 g, 96%) as a white solid. $R_f$0.79 (cyclohexane/$Et_2O$ 5:1); mp: 60 ˚C; $^1$H NMR (300 MHz, $CDCl_3$) δ 7.82 (t, *J* = 7.9 Hz, 1H), 8.11 (s, 1H), 8.22 (d, *J* = 7.3 Hz, 1H), 8.46 (d, *J* = 8.6 Hz, 1H), NMR data were in agreement with the lit.:[13]; $^{13}$C NMR (125 MHz, $CDCl_3$) δ 120.9 (q, *J* = 276.0 Hz), 122.0 (q, *J* = 2.0 Hz), 123.6 (q, *J* = 273.8 Hz), 128.9, 129.4, 129.8 (q, *J* = 30.8 Hz), 130.5 (q, *J* = 5.3 Hz), 131.5, 138.5, 144.5, 148.6 (q, *J* = 36.1 Hz); IR $U_{max}$=1577, 1422, 1302, 1136, 1098, 1010, 876, 824 $cm^{-1}$; GCMS (*m*/*z*): 343; HRMS calcd for $C_{11}H_4BrF_6NNa$ (M+Na)+365.9329, found 365.9346.

**2,8-bis(trifluoromethyl)quinolin-4-yl trifluoromethanesulfonate (6b)**

To a solution of **5** (1.5 g, 5.34 mmol) in toluene/aqueous solution of LiOH (5%, w/v) (22 mL; 1:1, v/v) was added dropwise anhydride triflique (1.1 mL, 6.41 mmol), at 0 ˚C. The resulting mixture was allowed to warm to room temperature and was stirred during 3 h. The reaction mixture was washed with water and the organic phase was dried over anhydrous sodium sulfate, and was concentrated under reduced pressure to afford **6b** (1.12 g, 50 %) as a colorless oil. $^1$H NMR (300 MHz, $CDCl_3$) δ 7.86 (s, 1H), 7.95 (t, *J* = 8.0 Hz, 1H), 8.34 (d, *J* = 7.8 Hz, 1H), 8.37 (d, *J* = 7.8 Hz, 1H); $^{13}$C NMR (75 MHz, $CDCl_3$) δ 109.4 (q, *J* = 1.7 Hz), 118.9 (q, *J* = 320.5 Hz), 120.4 (q, *J* = 275.5 Hz), 122.0 (q, *J* = 273.6 Hz), 125.1, 129.1(q, *J* = 27.3 Hz), 129.4, 130.8 (q, *J* = 5.0 Hz), 145.6, 149.4 (q, *J* = 36.8 Hz), 154.0.

**2,8-bis(trifluoromethyl)quinolin-4-yl 4-methylbenzenesulfonate (6c)**

To a solution of **5** (500 mg, 1.78 mmol) in acetone (4 mL) was added an aqueous solution of NaOH 2N until pH=11, and *p*-toluenesulfonyl chloride (680 mg, 3.56 mmol) at 0 ˚C. The reaction mixture was stirred overnight. After allowing warming to room temperature, the solvent was reduced under pressure. The resulting residue was washed with water and filtered to afford compound **6c** (746 mg, 96 %) as a white solid. mp: 107 ˚C; $^1$H NMR (300 MHz, $CDCl_3$) δ 7.86 (s, 1H), 7.95 (t, *J* = 7.9 Hz, 1H), 8.34 (d, *J* = 7.8 Hz, 1H), 8.37 (d, *J* = 7.8 Hz, 1H); $^{13}$C NMR (75 MHz, $CDCl_3$) δ 21.7, 109.4, 120.6 (q, *J* = 275.6 Hz), 123.2 (q, *J* = 273.8 Hz), 123.6, 126.1, 127.9, 130.0 (q, *J* = 5.2 Hz), 130.4, 131.4 (q, *J* = 3.9 Hz), 145,3, 147.0, 149.1 (q, *J* = 36.0 Hz), 154.6; HRMS calcd for $C_{18}H_{11}F_6NO_3SNa$ (M+Na)+ 458.0262,

found 458.0270.

**2,8-bis(trifluoromethyl)-4-vinylquinoline (3)**

*Method A:* To a solution of **6a** (100 mg, 0.29 mmol), TBAB (93 mg, 0.29 mmol), $K_2CO_3$ (80 mg, 0.58 mmol), and $Pd(PPh_3)_2Cl_2$ (20 mg, 10 mol %) in dry MeCN (580 μL), was added tributylvinyltin (102 μL, 0.35 mmol), under argon atmosphere. The tube was scelled and the mixture was stirred at 90 °C. After 4 h, the reaction was allowed to cool to room temperature then was filtered through a pad of celite and washed with MeCN. The filtrate was evaporated under reduced pressure and the residue was purified by column chromatography (cyclohexane/$Et_2O$ 5:1) to afford **3** (64 mg, 75%) as a white solid. $R_f$ 0.34 (cyclohexane/$Et_2O$ 5:1); mp: 76 °C; $^1H$ NMR (300 MHz, $CDCl_3$) δ 5.83 (dd, *J* = 11.1, 0.7 Hz, 1H), 6.09 (dd, *J* = 17.3, 0.7 Hz, 1H), 7.43 (dd, *J* = 17.3, 11.1 Hz, 1H), 7.72 (t, *J* = 7.9 Hz, 1H), 7.86 (s, 1H), 8.15 (d, *J* = 6.9 Hz, 1H), 8.33 (d, *J* = 8.6 Hz, 1H); $^{13}C$ NMR (75 MHz, $CDCl_3$) δ 114.1 (q, *J* = 2.1 Hz), 121.3 (q, *J* = 275.5 Hz), 123.2, 123.6 (q, *J* = 273.6 Hz), 127.0, 127.9, 127.8, 129.0 (q, *J* = 5.5 Hz), 129.2 (q, *J* = 30.5 Hz), 131.0, 144.0, 146.2, 148.4 (q, *J* = 35.6 Hz), NMR data were in agreement with the lit.: [12]; IR $U_{max}$= 1590, 1426, 1303, 1134, 1108, 888, 833 $cm^{-1}$; GCMS (*m*/*z*): 291; HRMS calcd for $C_{13}H_7F_6NNa$ (M+Na)+314.0380, found 314.0390.

*Method B:* To a solution of **6a** (1.16 mmol) in toluene/aqueous solution of KOH (4M) (9 mL; 8:1, v/v) was added dibutyl vinylboronate (1.28 mmol) and $Pd(PPh_3)_4$ (0.035 mmol), under under nitrogen. The reaction mixture was stirred at reflux during 24 h. The reaction was allowed to cool to room temperature, transferred to a separating funnel and then was washed with water (20 mL) and toluene (2 x 20 mL), the washings being added to the separating funnel. The organic layer was separated, washed with an aqueous 1 M NaOH solution, then with a brine solution, dried over $Na_2SO_4$, and evaporated to dryness. The residue was purified by column chromatography (cyclohexane/ether 5:1) to afford **3** (82%) as a white solid.

*Method C*: To a suspension of potassium vinyltrifluoroborate (1.16 mmol), cesium carbonate (3.5 mmol), $PdCl_2$ (dppf)·$CH_2Cl_2$ (0.116 mmol), and **6a** (1.16 mmol) in THF (15 mL) was added water

(1.5 mL), under a nitrogen atmosphere. The reaction mixture was stirred at reflux during 24 h. The reaction was allowed to cool to cool to room temperature, diluted with water (25 mL), and was extracted with diethyl ether (3 x 25 mL). The combined organic extracts were washed with brine and then dried over $Na_2SO_4$, filtered and was concentred *in vacuo*. The residue was purified by column chromatography (cyclohexane/ether 5:1) to afford **3** (71%) as a white solid.

**(*R*)-1-[2,8-bis(trifluoromethyl)quinolin-4-yl]ethane-1,2-diol (4a)**

To a solution of AD-mix-β (4.8 g) and $K_2OsO_2(OH)_4$ (12.7 mg, 1 mol %) in *t*-BuOH/$H_2O$ (34.4 mL; 1:1 v/v) was added **3** (1 g, 3.44 mmol) at 0 °C and stirred overnight. The reaction was quenched at 0 °C by addition of $Na_2SO_3$ (5.16 g), then was warmed to room temperature and stirred for 30 min. The reaction mixture was extracted with EtOAc, dried with anhydrous $Na_2SO_4$ and evaporated under reduced pressure. The residue was precipitated in cyclohexane, filtered on buchner to afford **4a** (945 mg, 84%, GCMS: 100%, and 98% ee) as a white solid. HPLC analysis (Chiralpak IB column, heptane/*i*-PrOH, 95:5; flow 1.0 mL/min, $t_r$(*R*) = 26.8 min, $t_r$(*S*) = 32.9 min); $[\alpha]_D^{20}$ -52° (*c* 0.25, DCM); mp: 130-135 °C; $^1H$ NMR (300 MHz, $CDCl_3$) δ 3.77 (dd, *J* = 11.6, 4.2 Hz, 1H), 3.89 (dd, *J* = 11.6, 5.2 Hz, 1H), 5.62 (dd, *J* = 5.9, 4.4 Hz, 1H), 7.83 (t, *J* = 7.9 Hz, 1H), 8.15 (s, 1H), 8.23 (d, *J* = 7.2 Hz, 1H), 8.54 (d, *J* = 8.5 Hz, 1H); $^{13}C$ NMR (75 MHz, $CDCl_3$) δ 67.9, 71.7,116.3 (q, *J* = 2.1 Hz), 122.9 (q, *J* = 274.8 Hz), 125.1 (q, *J* = 272.8 Hz), 128.4, 129.3, 130.0 (q, *J* = 5.6 Hz), 130.1 (q, *J* = 29.7 Hz), 144.8, 149.2 (q, *J* = 34.9 Hz), 153.4; IR $U_{max}$= 3266, 2937, 1605, 1587, 1308, 1128, 1102, 838 $cm^{-1}$; GCMS (*m*/*z*): 325; HRMS calcd for $C_{13}H_9F_6NO_2Na$ (M+Na)+348.0435, found 348.0420.

**(*S*)-1-[2,8-bis(trifluoromethyl)quinolin-4-yl]ethane-1,2-diol (4b)**

According to the same procedure that **4a,** the product **4b** was prepared starting from **3** (640 mg) to obtain **4b** (558 mg, 78%, 97% ee) as a white solid. HPLC analysis (Chiralpak IB column, heptane/*i*-PrOH, 95:5; flow 1.0 mL/min, $t_r$(*R*) = 26.8 min, $t_r$(*S*) = 31.8 min); mp: 130-135 °C; [α]$D^{20}$ +62 (*c* 0.25, DCM); $^1H$ NMR (300 MHz, $CDCl_3$) δ 3.77 (dd, *J* = 11.6, 4.2 Hz, 1H), 3.89 (dd, *J* = 11.6, 5.2 Hz, 1H), 5.62 (dd, *J* = 5.9, 4.4 Hz, 1H,), 7.83 (t, *J* = 7.9 Hz, 1H), 8.15 (s, 1H), 8.23 (d, *J* = 7.2 Hz, 1H), 8.54 (d, *J* = 8.5 Hz, 1H); $^{13}C$ NMR (75 MHz, $CDCl_3$) δ 67.9, 71.7, 116.3 (q, *J* = 2.1 Hz), 122.9 (q, *J* = 274.8 Hz), 125.1 (q, *J* = 272.8 Hz), 128.4, 129.3, 130.0 (q, 1H, *J* = 5.6 Hz), 130.1 (q, *J* = 29.7 Hz), 144.8, 149.2 (q, *J* = 34,9 Hz), 153,4; IR $U_{max}$= 3266, 2937, 1605, 1587, 1308, 1128, 1102, 838 $cm^{-1}$; GCMS (*m*/*z*): 325; HRMS calcd for $C_{13}H_9F_6NO_2Na$ $(M+Na)^+$348.0435, found 348.0440.

**(*R*)-1-(2,8-bis(trifluoromethyl)quinolin-4-yl)-2-((*tert*-butyldimethylsilyl)oxy)ethanol (7)**

To a solution of **4a** (140 mg, 0.43 mmol) in dry DMF (5 mL), under argon atmosphere, was added TDBMSCI (64.8 mg, 0.43 mmol), and imidazole (29.2 mg, 0.43 mmol). The mixture was stirred at room temperature for 24 h. The

solution was concentrated under reduced pressure, and then the residue was dissolved in EtOAc and was washed with water, dried over $Na_2SO_4$, filtered and concentrated *in vacuo.* The residue was purified by column chromatography (DCM/MeOH 99:1) to afford **7** (74 mg, 39%, 99% ee) as a colorless oil. HPLC analysis (Chiralpak IB column, heptanes/*i*-PrOH 99:1; flow 1 mL/min, $t_r(R)$ = 13.9 min, $t_r(S)$ = 16.6 min); [α]D$^{21}$ -41.1 (*c* 0.25, DCM); $R_f$ 0.88 (DCM/MeOH 99:1); $^1H$ NMR (300 MHz, $CDCl_3$) δ 0.03 (s, 3H), 0.06 (s, 3H), 0.90 (s, 9H), 3.67 (dd, *J* = 10.3, 7.4 Hz, 1H), 4.03 (dd, *J* = 10.3, 3.7 Hz, 1H), 5.57 (dd, *J* = 7.2, 3.4 Hz, 1H), 7.74 (t, *J* = 8.0 Hz, 1H), 8.10 (s, 1H), 8.15 (d, *J* = 7.2 Hz, 1H), 8.24 (d, *J* = 8.2 Hz, 1H); $^{13}C$ NMR (75 MHz, $CDCl_3$) 18.2, 25.8, 67.5, 70.3, 115.2 (q, *J* = 2.05 Hz), 121.3 (q, *J* = 275.1 Hz), 123.5 (q, *J* = 273.6 Hz), 126.8, 127.0, 127.2, 128.7 (q, *J* = 5.5 Hz), 129.2 (q, *J* = 32.7 Hz), 143.6, 148.5 (q, *J* = 36.8 Hz), 149.2; IR $U_{max}$= 2956, 2932, 2861, 1604, 1586, 1431, 1308, 1144, 1108, 837, 807 $cm^{-1}$; HRMS calcd for $C_{19}H_{23}F_6NO_2SiNa$ $(M+Na)^+$ 462.1300, found 462.1320.

**(*R*)-(*R*)-1-(2,8-bis(trifluoromethyl)quinolin-4-yl)-2-((*tert*-butyldimethylsilyl)oxy)ethyl-3,3,3-trifluoro-2-methoxy-2-phenylpropanoate (8a)**

To a solution of **7** (43 mg, 0,098 mmol) in dry DCM (1 mL), under argon atmosphere, was added at 0 °C (*R*) (+)-MTPA (27 mg, 0.12 mmol), EDCI (37.5 mg, 0.2 mmol), and DMAP (3.6 mg, 0.029 mmol). The mixture was stirred at room temperature for 24 h. The solution was concentrated under reduced pressure. The residue was purified by column chromatography (DCM/MeOH 99:1) to afford **8a** (38 mg, 58%, 95% ee) as a yellow oil. The ee was determined by $^1H$ NMR. $[\alpha]_D^{24}$ -19.2 (*c* 0.25, DCM); $R_f$ 0.82 (DCM/MeOH 99:1); $^1H$ NMR (600 MHz, $CDCl_3$) δ -0.044 (s, 3H), -0.041 (s, 3H), 0.81 (s, 9H), 3.65 (d, *J* = 0.68 Hz, 3H), 3.99 (dd, *J* = 11.4, 4.0 Hz, 1H), 4.05 (dd, *J* = 11.4, 6.8 Hz, 1H), 6.77 (dd, *J* = 6.8, 4.0 Hz, 1H), 7.49-7.36 (m, 5H), 7.57 (s, 1H), 7.77 (t, *J* = 7.9 Hz, 1H), 8.21 (d, *J* = 7.2 Hz), 8.32 (d, *J* = 8.4 Hz, 1H); $^{13}C$ NMR (150 MHz, $CDCl_3$) δ -5.6 (2C), 18.1, 25.6, 55.8, 65.1, 74.3, 115.4 (q, *J* = 1.8 Hz), 120.9 (q, *J* = 275.6 Hz), 123.2 (q, *J* = 288.7 Hz), 123.4 (q, *J* = 273.7 Hz), 126.4, 126.9 (3C), 127.7, 128.6, 129.1 (q, *J* = 5.1 Hz), 129.7 (q, *J* = 30.4 Hz), 130.1, 131.7, 143.8, 145.0, 148.2 (q, *J* = 35.5 Hz), 165.9; IR $U_{max}$= 2955, 2930, 2857, 1755, 1605, 1312, 1147, 1112, 1018, 837 $cm^{-1}$; HRMS calcd for $C_{29}H_{30}F_9NO_4SiNa$ (M+Na)+678.1698, found 678.1688.

**(*S*)-(*R*)-1-(2,8-bis(trifluoromethyl)quinolin-4-yl)-2-((*tert*-butyldimethylsilyl)oxy)ethyl-3,3,3-trifluoro-2-methoxy-2-phenylpropanoate (8b)**

According to the same procedure that **8a,** the product **8b** was prepared starting from **7** (41 mg) to obtain **8b** (30 mg, 49%, 98% ee) as a yellow oil. The ee was determined by $^1H$ NMR. $[\alpha]_D^{24}$ -36.7 (*c* 0.25, DCM); $R_f$ 0.82 (DCM/MeOH 99:1); $^1H$ NMR (600 MHz, $CDCl_3$) δ -0.12 (s, 3H), -0.11(s, 3H), 0.74 (s, 9H), 3.49 (d, *J* = 1.2 Hz, 3H), 3.98 (dd, *J* = 11.2, 4.6 Hz, 1H), 4.02 (dd, *J* = 11.1, 5.9 Hz, 1H), 6.80 (t, *J* = 5.19 Hz, 1H), 7.57-7.32 (m, 5H), 7.81-7.78 (m, 2H), 8.22 (d, *J* = 7.2 Hz, 1H), 8.37 (d, *J* = 8.5 Hz, 1H); $^{13}C$ NMR (150 MHz, $CDCl_3$) δ -5.9, -5.8, 18.0, 25.5, 55.3, 65.0, 73.9,115.8 (q, *J* = 1.9 Hz), 121.0 (q, *J* = 275.6 Hz), 123.3 (q, *J* = 289.0 Hz), 123.4 (q, *J* = 273.8 Hz), 126.5, 127.2, 127.4, 127.7, 128.6, 129.2 (q, *J* = 5.6 Hz), 129.7 (q, *J* = 30.7 Hz), 129.9, 131.4, 143.8, 145.4, 148.1 (q, *J* = 35.5 Hz), 165.9; IR $U_{max}$= 2955, 2930, 2857, 1755, 1605, 1312, 1147, 1112, 1018, 837 $cm^{-1}$; HRMS calcd for $C_{29}H_{30}F_9NO_4SiNa$ $(M+Na)^+$ 678.1698, found 678.1688.

**(R)-4-(oxiran-2-yl)-2,8-bis(trifluoromethyl)quinoline (2a)**

To a solution of **4a** (800 mg, 0.62 mmol) in dry DCM (8.8 mL), under argon atmosphere, was added trimethylorthoacetate (236 μL, 1.85 mmol) and PTSA (5.3 mg, 0.031 mmol). The mixture was stirred for 4h at room temperature. The volatiles were removed *in vacuo,* and the flask pumped on high vacuum to remove excess MeOH. The residue was redissolved in dry DCM (8.8 mL), under argon, and was cooled to 0 °C. Trimethylsilyl chloride (234 μL, 1.85 mmol) was added dropwise. The reaction was warmed to room temperature and was stirred overnight. The volatiles were removed *in vacuo*, and the residue was redissolved in MeOH (8.8 mL). $K_2CO_3$ (254.8 mg, 1.85 mmol) was added, and the mixture was stirred for 5h. The reaction was poured onto saturated aqueous solution of $NH_4Cl$, extracted with DCM. The organic layer was dried over anhydrous $Na_2SO_4$, filtered, and concentrated under reduced pressure. The crude was purified by column chromatography (cyclohexane/$Et_2O$ 1:1), to afford **2a** (513 mg, 68%, 92% ee) as a yellow solid. HPLC analysis (Chiralpak IB column, heptane/*i*-PrOH, 99:1; flow 1.0 mL/min, $t_r$(S) = 15.1 min, $t_r$R) = 17.4 min); [α]D$^{20}$ -52 (*c* 0.25, DCM); $R_f$ 0.36 (cyclohexane/$Et_2O$ 1:1); mp: 84 °C; $^1H$ NMR (300 MHz, $CDCl_3$) δ 2.83 (dd, *J* = 5.7, 2.5 Hz, 1H), 3.42 (dd, *J* = 5.7, 4.2 Hz, 1H), 4.55 (dd, *J* = 3.6, 2.7 Hz, 1H), 7.79 (t, *J* = 7.9 Hz, 1H), 7.81 (s, 1H), 8.20 (d, *J* = 7.2 Hz, 1H), 8.39 (d, *J* = 8.4 Hz, 1H); $^{13}C$ NMR (150 MHz, $CDCl_3$): 49.2, 50.8, 113.3 (q, *J* = 2.0 Hz), 121.1 (q, *J* = 275.5 Hz), 123.4 (q, *J* = 273.6 Hz), 127.0, 127.4, 127.6, 129.1 (q, *J* = 5.5 Hz), 129.4 (q, *J* = 30.5 Hz), 143.3, 146.8, 148.7 (q, *J* = 35.5 Hz), 149.5, NMR data were in agreement with the lit.:[12]; IR $U_{max}$= 1606, 1587, 1431, 1308, 1276, 1213, 1102, 1069, 895, 982, 822 $cm^{-1}$; GCMS: 307; HRMS calcd for $C_{13}H_7F_6NONa$ $(M+Na)^+$, 330.0330 found 330.0314.

**(*S*)-4-(oxiran-2-yl)-2,8-bis(trifluoromethyl)quinoline (2b)**

According to the same procedure that **2a,** the product **2b** was prepared starting from **4b** (800 mg) to obtain **2b** (437 mg, 57%, 96% ee) as a yellow solid. HPLC analysis (Chiralpak IB column, heptane/i-PrOH, 99:1; flow 1.0 mL/min, $t_r(S)$ = 14.9 min, $t_r(R)$ = 17.4 min); [α]D$^{20}$ +45 (*c* 0.25, DCM); $R_f$ 0.36 (cyclohexane/$Et_2O$ 1:1); mp: 84 ˚C; $^1H$ NMR (300 MHz, $CDCl_3$) δ 2.83 (dd, *J* = 5.7, 2.5 Hz, 1H), 3.42 (dd, *J* = 5.7, 4.2 Hz, 1H), 4.55 (dd, *J* = 3.6, 2.7 Hz, 1H), 7.79 (m, 2H), 8.20 (d, *J* = 7.2 Hz, 1H), 8.39 (d, *J* = 8.4 Hz, 1H); $^{13}C$ NMR (150 MHz, $CDCl_3$): 49.2, 50.8, 113.3 (q, *J* = 2.0 Hz), 121.1 (q, *J* = 275.5 Hz), 123.4 (q, *J* = 273.6 Hz), 127.0, 127.4, 127.6, 129.1 (q, *J* = 5.5 Hz), 129.4 (q, *J* = 30.5 Hz), 143.3, 146.8, 148.7 (q, *J* = 35.5 Hz), 149.5, NMR data were in agreement with the lit.:[12]; IR $U_{max}$= 1606, 1587, 1431, 1308, 1276, 1213, 1102, 1069, 895, 982, 822 $cm^{-1}$; GCMS: 307; HRMS calcd for $C_{13}H_7F_6NONa$ $(M+Na)^+$, 330.0330 found 330.0314.

**Example 2 General method for preparation of amino-alcohol 1**

To a solution of **2a** or **2b** (50 mg, 0.16 mmol) in *i*-PrOH (3.2 mL) was added the appropriate amine (0.81 mmol). The mixture was stirred for 24 h at 90 ˚C. The volatiles were removed *in vacuo*. The crude was then purified by column chromatography. In the following examples, only a part of the compounds according to the invention is presented. However, the invention is not limited to such compositions as far as the composition fall within the scope of the present claims.

**(*R*)-2-(benzylamino)-1-(2,8-bis(trifluoromethyl)quinolin-4-yl)ethanol (1a)**

The crude was purified by column chromatography (EtOAc/MeOH/$NH_4OH$ 90:5:5), to afford **1a** (44 mg, 66%, 95% ee) as a white solid. HPLC analysis (Chiralpak IB column, heptane/EtOH, 90:10; flow 0.5 mL/min, $t_r$(R) = 16.8 min, $t_r$(S) = 18.9 min); [α]D$^{27}$ -34.5 (*c* 0.25, DMSO); $R_f$ 0.78 (EtOAc/MeOH/$NH_4OH$ 80:10:10); mp: 163 ˚C; $^1H$ NMR (300 MHz, $CDCl_3$) δ2.78 (dd, *J* = 12.4, 6.9 Hz, 1H), 2.88 (dd, *J* = 12.5, 3.9 Hz, 1H), 3.72, (s, 2H), 5.61 (m, 1H), 7.20 (m, 5H), 7.85 (t, *J* = 8.0 Hz, 1H), 8.12 (s, 1H), 8.32 (d, *J* = 7.3Hz, 1H), 8.55 (d, *J* = 8.6 Hz, 1H); $^{13}C$ NMR (150 MHz, $CDCl_3$) δ 52.3, 55.4, 68.1, 114.7, 121.5 (q, *J* = 275.4 Hz), 123.3 (q, *J* = 273.1 Hz), 126.4, 126.7, 126.9 (q, *J* = 28.5 Hz), 127.4, 127.7, 127.9, 129.1, 129.4 (q, *J* = 5.5 Hz), 140.5, 142.5, 146.6 (q, *J* = 34.1 Hz), 155.0; IR $U_{max}$= 1603, 1587, 1430, 1310, 1300, 1127, 1106, 903, 881, 835 $cm^{-1}$; HRMS calcd for $C_{20}H_{17}F_6N_2O$ (M+H)+ 415.1245, found 415.1265.

**(*S*)-2-(benzylamino)-1-(2,8-bis(trifluoromethyl)quinolin-4-yl)ethanol (1f)**

The crude was purified by column chromatography (EtOAc/MeOH/$NH_4OH$ 90:5:5), to afford **1f** (59 mg, 89%, 98% ee) as a white solid. HPLC analysis (Chiralpak IB column, heptane/EtOH, 90:10; flow 0.5 mL/min, $t_r(R)$ = 16.8 min, $t_r(S)$ = 18.9 min); [α]D$^{27}$ +46.3 (*c* 0.25, DMSO); $R_f$ 0.78 (EtOAc/MeOH/$NH_4OH$ 80:10:10); mp: 163 ˚C; $^1H$ NMR (300 MHz, $CDCl_3$) δ 2.78 (dd, *J* = 12.4, 6.9 Hz, 1H), 2.88 (dd, *J* = 12.5, 3.9 Hz, 1H), 3.72, (s, 2H), 5.61 (m, 1H), 7.20 (m, 5H), 7.85 (t, *J* = 8.0 Hz, 1H), 8.12 (s, 1H), 8.32 (d, *J* = 7.3Hz, 1H), 8.55 (d, *J* = 8.6 Hz, 1H); $^{13}C$ NMR (150 MHz, $CDCl_3$) δ 52.3, 55.4, 68.1, 114.7, 121.5 (q, *J* = 275.4 Hz), 123.3 (q, *J* = 273.1 Hz), 126.4, 126.7, 126.9 (q, *J* = 28.5 Hz), 127.4, 127.7, 127.9, 129.1, 129.4 (q, *J* = 5.5 Hz), 140.5, 142.5, 146.6 (q, *J* = 34.1 Hz), 155.0; IR $U_{max}$= 1603, 1587, 1430, 1310, 1300, 1127, 1106, 903, 881, 835 $cm^{-1}$; HRMS calcd for $C_{20}H_{17}F_6N_2O$ $(M+H)^+$415.1245, found 415.1265.

**(*R*)-ethyl-4-(4-(2-(2,8-bis(trifluoromethyl)quinolin-4-yl)-2-(hydroxyethyl)piperazin-1-yl)butanoate (1 b)**

The crude was purified by column chromatography (DCM/MeOH 90:10), to afford 1b (29 mg, 35%, 89% ee) as a yellow oil. HPLC analysis (Chiralpak IB column, heptanes/*i*-PrOH/EDA 90:10:0.1; flow 1 mL/min, $t_r(R)$ = 9.0 min, $t_r(S)$ = 20.6 min); $[\alpha]_D^{24}$ -70.7 (*c* 0.25, DCM); $R_f$ 0.43 (DCM/MeOH 90:10); $^1H$ NMR (300 MHz, $CDCl_3$) δ 1.26 (t, *J* = 7.1 Hz, 3H), 1.88-1.78 (m, 2H), 2.85-2.26 (m, 14H), 4.13 (q, *J* = 7.2 Hz, 2H), 5.54 (dd, *J* = 10.5, 3.2 Hz, 1H), 7.72 (t, *J* = 7.1 Hz, 1H), 8.12-8.14 (m, 2H); $^{13}C$ NMR (150 MHz, $CDCl_3$) δ14.2, 22.1,32.2, 53.0,57.5, 60.3, 64.5, 65.1,114.5 (q, *J* = 1.8 Hz), 121.2 (q, *J* = 275.6 Hz), 123.5 (q, *J* = 273.9), 126.6, 126.,7, 127.0, 128.7 (q, *J* = 5.2 Hz), 129.6 (q, *J* = 29.8Hz), 143.6, 148.7 (q, *J* = 34.9 Hz), 151.1, 173.5; IR $U_{max}$= 2941, 2818, 1731, 1604, 1585, 1430, 1308, 1140, 1108, 907 $cm^{-1}$; HRMS calcd for $C_{23}H_{28}F_6N_3O_3$ (M+H)+ 508.2035, found 508.2056.

**(*S*)-ethyl-4-(4-(2-(2,8-bis(trifluoromethyl)quinolin-4-yl)-2-(hydroxyethyl)piperazin-1-yl)butanoate (1g)**

The crude was purified by column chromatography (DCM/MeOH 90:10), to afford **1g** (31 mg, 38%, 97% ee) as a yellow oil. HPLC analysis (Chiralpak IB column, heptanes/ *i*-PrOH/EDA 90:10:0.1; flow 1 mL/min, $t_r(R)$ =9.1 min, $t_r(S)$ = 20.1 min); [α]D$^{23}$ +78.2 (c 0.25, DCM); $R_f$ 0.43 (DCM/MeOH 90:10); $^1H$ NMR (300 MHz, $CDCl_3$) δ 1.26 (t, *J* =

7.1 Hz, 3H), 1.88-1.78 (m, 2H), 2.85-2.26 (m, 14H), 4.13 (q, *J* = 7.2 Hz, 2H), 5.54 (dd, *J* = 10.5, 3.2 Hz, 1H), 7.72 (t, *J* = 7.1 Hz, 1H), 8.12-8.14 (m, 2H); $^{13}C$ NMR (150 MHz, $CDCl_3$) δ 14.2, 22.1, 32.2, 53.0, 57.5, 60.3, 64.5, 65.1, 114.5 (q, *J* = 1.8 Hz), 121.2 (q, *J* = 275.6 Hz), 123.5 (q, *J* = 273.9), 126.6, 126.,7, 127.0, 128.7 (q, *J* = 5.2 Hz), 129.6 (q, *J* = 29.8Hz), 143.6, 148.7 (q, *J* = 34.9 Hz), 151.1, 173.5; IR $U_{max}$= 2941, 2818, 1731, 1604, 1585, 1430, 1308, 1140, 1108, 907 $cm^{-1}$; HRMS calcd for $C_{23}H_{28}F_6N_3O_3$ (M+H)+ 508.2035, found 508.2056.

**(*R*)-1-(2,8-bis(trifluoromethyl)quinolin-4-yl)-2-(pentylamino)ethanol (1c)**

The crude was purified by column chromatography (DCM/MeOH 90:10), to afford **1c** (56 mg, 88%, 94% ee) as a white solid. HPLC analysis (Chiralpak IB column, heptane/i-PrOH/EDA, 99:1:0.1; flow 1 mL/min, $t_r$(R) = 21.5 min, $t_r$(S) = 25.3 min); [α]D$^{26}$ -50.3 (*c* 0.25, MeOH); $R_f$ 0.53 (DCM/MeOH 90:10); mp: 120 ˚C; $^1H$ NMR (300 MHz, MeOD) δ 0.92 (t, *J* = 6.5 Hz, 3H), 1.36-1.21 (m, 4H), 1.65-1.47 (m, 2H), 2.72-2.64 (m, 2H), 2.83 (dd, *J* = 12.30, 9.32 Hz, 1H), 2.99 (dd, *J* = 12.63, 2.20 Hz, 1H), 5.68 (dd, *J* = 9.0, 2.0 Hz, 1H), 7.87 (t, *J* = 7.9 Hz, 1Hz), 8.15 (s, 1H), 8.26 (d, *J* = 7.0 Hz, 1H), 8.53 (d, *J* = 8.6 Hz, 1H); $^{13}C$ NMR (150 MHz, MeOD) δ 14.4, 23.6, 30.2, 30.6, 50.5, 57.2, 69.4, 115.7 (q, *J* = 1.96 Hz), 122.9 (q, *J* = 274.6 Hz), 125.1 (q, *J* = 272.6 Hz),128.0, 128.8, 129.1, 130.3 (q, *J* = 60.1 Hz), 130.3 (q, *J* = 5.7 Hz), 144.9, 149.4 (q, *J* = 34.8 Hz), 154.7; IR $U_{max}$= 2934, 2841, 2357, 1604, 1586, 1432, 1303, 1119, 1104, 889, 835 $cm^{-1}$; HRMS calcd for $C_{18}H_{21}F_6N_2O$ (M+H)+ 395.1558, found 395.1544.

**(*S*)-1-(2,8-bis(trifluoromethyl)quinolin-4-yl)-2-(pentylamino)ethanol (1h)**

The crude was purified by column chromatography (DCM/MeOH 90:10), to afford **1h** (56 mg, 93%, 99% ee) as a white solid. HPLC analysis (Chiralpak IB column, heptane/i-PrOH/EDA, 99:1:0.1; flow 1 mL/min, $t_r(R)$ = 22.2 min, $t_r(S)$ = 25.3 min); $[\alpha]_D^{26}$ +49.8 (*c* 0.25, MeOH); $R_f$ 0.53 (DCM/MeOH 90:10); mp: 120 ˚C; $^1H$ NMR (300 MHz, MeOD) δ 0.92 (t, *J* = 6.5 Hz, 3H), 1.36-1.21 (m, 4H), 1.65-1.47 (m, 2H), 2.72-2.64 (m, 2H), 2.83 (dd, *J* = 12.30, 9.32 Hz, 1H), 2.99 (dd, *J* = 12.63, 2.20 Hz, 1H), 5.68 (dd, *J* = 9.0, 2.0 Hz, 1H), 7.87 (t, *J* = 7.9 Hz, 1Hz), 8.15 (s, 1H), 8.26 (d, *J* = 7.0 Hz, 1H), 8.53 (d, *J* = 8.6 Hz, 1H); $^{13}C$ NMR (150 MHz, MeOD) δ 14.4, 23.6, 30.2, 30.6, 50.5, 57.2, 69.4, 115.7 (q, *J* = 1.96 Hz), 122.9 (q, *J* = 274.6 Hz), 125.1 (q, *J* = 272.6 Hz),128.0, 128.8, 129.1, 130.3 (q, *J* = 60.1 Hz), 130.3 (q, *J* = 5.7 Hz), 144.9, 149.4 (q, *J* = 34.8 Hz), 154.7; IR $U_{max}$= 2934, 2841, 2357, 1604, 1586, 1432, 1303, 1119, 1104, 889, 835 $cm^{-1}$; HRMS calcd for $C_{18}H_{21}F_6N_2O$ (M+H)+ 395.1558, found 395.1544.

**(*R*)-1-(2,8-bis(trifluoromethyl)quinolin-4-yl)-2-(hexylamino)ethanol (1d)**

The crude was purified by column chromatography (DCM/MeOH, 90:10), to afford **1d** (58 mg, 88%, 97% ee) as a white solide. HPLC analysis (Chiralpak IB column, heptane/i-PrOH/EDA, 99:1:0.1 ; flow 1 mL/min, $t_r$(*R*) = 18.5 min, $t_r$(*S*) = 21.4 min); [α]D$^{26}$ -47.5 (*c* 0.25, MeOH); $R_r$ 0.58 (DCM/MeOH 90:10); mp: 108 ˚C; $^1H$ NMR (600 MHz, MeOD) δ 0.91 (t, *J* = 6.9 Hz, 3H), 1.43-1.42 (m, 6H), 1.62-1.49 (m, 2H), 2.76-2.59 (m, 2H), 2.82 (dd, *J* = 12.6, 9.1 Hz, 1H), 2.99 (dd, *J* = 12.6, 2.9 Hz, 1H), 5.68 (dd, *J* = 9.0, 2.5 Hz, 1H), 7.88 (t, *J* = 7.2 Hz, 1H), 8.15 (s, 1H), 8.26 (d, *J* = 8.6 Hz, 1H), 8.53 (d, *J* = 8.6 Hz); $^{13}C$ NMR (150 MHz, MeOD) δ 14.4, 23.7, 28.1, 30.5, 32.9, 50.5, 57.3, 69.4, 115.7 (q, *J* = 2.3 Hz), 122.9 (q, *J* = 274.4 Hz), 125.2 (q, *J* = 272.8 Hz), 128.0, 128.8, 129.1, 130.3 (q, *J* = 5.4 Hz), 130.3 (q, *J* = 30.3 Hz), 144.9, 149.4 (q, *J* = 35.8 Hz), 154.8; IR $U_{max}$= 2928, 2858, 1603, 1586, 1432, 1304, 1119, 1103, 886, 837 $cm^{-1}$; HRMS calcd for $C_{19}H_{22}F_6N_2O$ (M+H)+ 409.1726, found 409.1732.

**(*S*)-1-(2,8-bis(trifluoromethyl)quinolin-4-yl)-2-(hexylamino)ethanol (1i)**

The crude was purified by column chromatography (DCM/MeOH, 90:10), to afford **1f** (56mg, 85%, 97% ee) as a white solide. HPLC analysis (Chiralpak IB column, heptane/i-PrOH/EDA, 99:1:0.1 ; flow 1 mL/min, $t_r$(*R*) = 19.1 min, $t_r(S)$ = 21.4 min); [α]D$^{27}$ +54.0 (*c* 0.25, MeOH); $R_f$ 0.58 (DCM/MeOH 90:10); mp: 108 ˚C; $^1H$ NMR (600 MHz, MeOD) δ 0.91 (t, *J* = 6.9 Hz, 3H), 1.43-1.42 (m, 6H), 1.62-1.49 (m, 2H), 2.76-2.59 (m, 2H), 2.82 (dd, *J* = 12.6, 9.1 Hz, 1H), 2.99 (dd, *J* = 12.6, 2.9 Hz, 1H), 5.68 (dd, *J* = 9.0, 2.5 Hz, 1H), 7.88 (t, *J* = 7.2 Hz, 1H), 8.15 (s, 1H), 8.26 (d, *J* = 8.6 Hz, 1H), 8.53 (d, *J* = 8.6 Hz); $^{13}C$ NMR (150 MHz, MeOD) δ 14.4, 23.7, 28.1, 30.5, 32.9, 50.5, 57.3, 69.4, 115.7 (q, *J* = 2.3 Hz), 122.9 (q, *J* = 274.4 Hz), 125.2 (q, *J* = 272.8 Hz), 128.0, 128.8, 129.1, 130.3 (q, *J* = 5.4 Hz), 130.3 (q, *J* = 30.3 Hz), 144.9, 149.4 (q, *J* = 35.8 Hz), 154.8; IR $U_{max}$= 2928, 2858, 1603, 1586, 1432, 1304, 1119, 1103, 886, 837 $cm^{-1}$; HRMS calcd for $C_{19}H_{22}F_6N_2O$ (M+H)+ 409.1726, found 409.1732.

**(*R*)-*tert*-butyl-(3-(4-(3((2-(2,8-bis(trifluoromethyl)quinolin-4-yl)-2-(hydroxyethyl)amino)propyl)piperazin-1-yl) propyl)carbamate (1e)**

The crude was purified by column chromatography (EtOAc/MeOH/$NH_4OH$ 80:10:10), to afford **1e** (88mg, 90%,

93% ee) as a colorless oil. HPLC analysis (Chiralpak IB column, MtBE/EDA 100:0.3; flow 1 mL/min, $t_r(R)$ = 6.2 min, $t_r$ *(S)* = 7.2 min); [α]D$^{24}$ -49.6 (*c* 0.25, DCM); $R_f$ 0.29 (EtOAc/MeOH/$NH_4$O 80:10:10); $^1$H NMR (300 MHz, MeOD) δ 1.38 (s, 9H), 1.64-1.59 (m, 2H), 1.67 (dt, *J* = 13.40, 6.76 Hz, 2H), 2.51-2.24 (m, 12H), 2.85-2.57 (m, 2H), 2.72 (dd, *J* = 12.41, 9.11 Hz, 1H), 3.05 (dd, *J* = 12.51, 2.77 Hz, 1H), 3.11 (m, 1H), 5.52 (dd, *J* = 8.4, 2.1 Hz, 1H), 7.67 (t, *J* = 7.9 Hz, 1H), 8.11-8.09 (m, 2H), 8.23 (d, *J* = 8.5 Hz, 1H); $^{13}$C NMR (150 MHz, MeOD) δ 26.2, 26.5, 28.3, 39.8, 48.0, 52.8, 53.2, 55.8, 56.5, 56.6, 67.7, 78.8, 114.5 (q, *J* = 1.6 Hz), 121.2 (q, *J* = 275.6 Hz), 123.4 (q, *J* = 273.8 Hz), 126.5, 126.9, 127.0, 128.6 (q, *J* = 5.6 Hz), 129.3 (q, *J* = 29.9 Hz), 143.5, 148.4 (q, *J* = 35.5 Hz), 152.0, 156.1; IR $U_{max}$= 2940, 1433, 1307, 1132, 1104, 885, 838 cm$^{-1}$; HRMS calcd for $C_{28}H_{40}F_6N_5O_3$ $(M+H)^+$608.3035, found 608.3035.

**(*S*)-*tert*-butyl-(3-(4-(3((2-(2,8-bis(trifluoromethyl)quinolin-4-yl)-2-(hydroxyethyl)amino)propyl)piperazin-1-yl) propyl)carbamate (1j)**

The crude was purified by column chromatography (EtOAc/MeOH/$NH_4$OH, 80:10:10), to afford **1j** (96mg, 98%, 92% ee) as a colorless oil. HPLC analysis (Chiralpak IB column, MtBE/EDA 100:0.3; flow 1 mL/min, $t_r(R)$ = 6.2 min, $t_r$ *(S)* = 7.2 min); [α]D$^{24}$ +50.6 (*c* 0.25, DCM); $R_f$ 0.29 (EtOAc/MeOH/$NH_4$OH 80:10:10); $^1$H NMR (300 MHz, MeOD) δ 1.38 (s, 9H), 1.64-1.59 (m, 2H), 1.67 (dt, *J* = 13.40, 6.76 Hz, 2H), 2.51-2.24 (m, 12H), 2.85-2.57 (m, 2H), 2.72 (dd, *J* = 12.41, 9.11 Hz, 1H), 3.05 (dd, *J* = 12.51, 2.77 Hz, 1H), 3.11 (m, 1H), 5.52 (dd, *J* = 8.4, 2.1 Hz, 1H), 7.67 (t, *J* = 7.9 Hz, 1H), 8.11-8.09 (m, 2H), 8.23 (d, *J* = 8.5 Hz, 1H); $^{13}$C NMR (150 MHz, MeOD) δ 26.2, 26.5, 28.3, 39.8, 48.0, 52.8, 53.2, 55.8, 56.5, 56.6, 67.7, 78.8, 114.5 (q, *J* = 1.6 Hz), 121.2 (q, *J* = 275.6 Hz), 123.4 (q, *J* = 273.8 Hz), 126.5, 126.9, 127.0, 128.6 (q, *J* = 5.6 Hz), 129.3 (q, *J* = 29.9 Hz), 143.5, 148.4 (q, *J* = 35.5 Hz), 152.0, 156.1; IR $U_{max}$= 2940, 1433, 1307, 1132, 1104, 885, 838 cm$^{-1}$; HRMS calcd for $C_{28}H_{40}F_6N_5O_3$ $(M+H)^+$ 608.3035, found 608.3035.

**Example 3 Biological tests**

**3-1. Antimalarial test**

Screenings of the compound according to the present invention (1f, 1h, 1i) and their isomers (1a, 1c, 1d)on strains of *Plasmodium falciparum* sensitive (3D7) and chloroquine resistant (W2) were realized.

The Clones of W2 and 3D7 were grown. 96-well plates with the test molecules at the final concentrations of 40000, 400, 200.100, 50, 25, 12.5, 6.35, 3.13, 1.56 and 0.78 nM in the growth medium were prepared. Melfloquine (MQ) at the same concentrations and chloroquine (CQ) at the concentrations adapted to each clone were used as the Controls. 8 negative controls were distributed on each plate.

Then, the molecules to be tested were diluted extemporaneously in the above preparation of the plates. The growth medium was deposited after drying plates. The study was initiated when the growth medium was at the ring stage and then stopped after 1 parasitic cycle (45h).

The activity was revealed by incorporation by parasites of tritiated hypoxanthine present in the growth medium. The activity was calculated by using the Pk-Fit software on all the experimental points obtained for each molecule (except the highest concentration). The results shown in table 5 are expressed in nM and correspond to calculate a 50% inhibitory concentration followed by un certainty about the value expressed in percentage.

Table 5. The biological effect of the compounds on *Plasmodium falciparum*

| Compound | W2 | 3D7 |
|---|---|---|
| 1 a | 41.1 (23.4%) | 104.5 (9.3%) |
| 1f | 19.1 (21.1%) | 35.1 (10.1%) |
| 1c | 38.2 (9.5%) | 74.7 (6.3%) |
| 1h | 6.98 (8.9%) | 8.33 (5.3%) |
| 1d | 142 (7.9%) | 205 (7.9%) |
| 1i | 9.40 (9.7%) | 14.5 (8.5%) |
| CQ | 572 (19.5%) | 32.5 (11.3%) |
| MQ | 26.5 (9.2%) | 52.2 (8.0%) |

The excellent antimalarial properties can be observed in the compounds according to the present invention notably in the compounds having (S)- enantiomer structure (1f, 1h, 1i) compared to the controls (CQ, MQ). The compounds having (R)- enantiomer (1a, 1c, 1d) also show their excellent antimalarial property.

### 3-2. Antibacterial test

The minimum inhibitory concentrations (MICs) were determined according to the recommendations of the Clinical and Laboratory Standards Institute (CLSI)[27]. The different strains were seeded in 96-well plates at a rate of $10^5$-$10^6$ colonies forming units per ml per well in Mueller-Hinton adjusted for cations ($Ca^{2+}$ 25 mg / L and $Mg^{2+}$ 12.5 mg / L). The different molecules to be tested were diluted to half in series to obtain final concentrations in wells ranging from 0.0625μg / mL to 128μg/ mL. After incubation for 18 to 24 h (48 h for *Enterococcus faecalis*) at 37˚C, the MIC was determined as the product concentration of the first well with no visual signs of bacterial growth (no disorder in the well). The results are shown in table 6, according to which excellent antibacterial properties can be observed in the compounds according to the present invention.

Table6. The antibacterial effects of the compounds

| Produit | *S. aureus* CIP[a] 103429 | *E. faecalis* CIP 103214 | *E. coli* DSM[b] 1103 | *P. aeruginosa* DSM 1117 | *SAAM 12* | *SAAM 27* | *SAAM 51* | *SAAM 64* | *SAAM 69* | *SAAM 86* | *SAAM 95* | *SAAM 98* |
|---|---|---|---|---|---|---|---|---|---|---|---|---|
| **Méfloquine** | 16-32 | 16-32 | 64 | >128 | 16 | 16 | 16 | 32 | 16 | 16 | 16 | 32 |
| **1a** | >128 | >128 | >128 | >128 | 4 | 4 | 4 | >128 | 8 | 8 | 4 | >128 |
| **1f** | 4-8 | 8 | >128 | >128 | 4 | 4 | 4 | >128 | 8 | 8 | 4 | 8 |
| **1h** | 16-32 | 16-32 | 16 | >128 | 8 | 8 | 8 | 16 | 8 | 16 | 8 | 16 |
| **1c** | 16-32 | 16-32 | 32 | >128 | 8 | 8 | 8 | 16 | 8 | 16 | 8 | 16 |
| **1i** | 4-8 | 4-8 | >128 | >128 | 2 | 2 | 2 | 4 | 4 | 8 | 2 | 8 |
| **1d** | 4-8 | 4-8 | 16 | >128 | 4 | 4 | 4 | 4 | 4 | 4 | 4 | 4 |
| **Ciprofloxacine** | 0,25 | 0,125-2 | 0,0625 | 0,0625 | 32 | 32 | >128 | 64 | 128 | 32 | 32 | 8 |

SAAM = *Staphylococcus aureus* Amiens (clinical strains resistant to methicillin)
[a]: Collection de l'institut Pasteur
[b]: Deutsche Sammlung von Mikroorganismen und Zellkulturen GmbH

REFERENCES

[1] WHO, 2007. http://www.who.int/tdr/diseases/malaria/direction.htm.
[2] Bhattacharjee, A. K.; Karle, J. M. J. Med. Chem. 2002, 39, 4622.
3 Go, M. L. Med. Res. Rev., 2003, 23, 456.
[4] Biot, C.; Chibale, K. Infect. Disorders-Drug Targets 2006, 6, 173.
[5] Karle, J. M.; Olmeda, R.; Gerena, L.; Milhous, W. K. Exp. Pharmacol. 1993, 76, 345.
[6] Phillips-Howard, P. A. and Ter Kuile, F. O. Drug Saf. 1995, 370.
[7] Weinke, T.; Trautmann, M.; Held, T.; Weber, G.; Eichenlaub, D.; Fleischer, K.; Kern, W.; Polhe, H. D. Am. J. Trop. Med. Hyg. 1991, 45, 86.
[8] Van Riemsdijk, M. M.; Van Der Klauw, M. M.; Van Heest, J. A. ; Reedeker, F. R.; Ligthelm, R. J.; Herings, R. M.; Stricker, B. H. Eur. J. Clin. Pharmacol. 1997, 52, 1.
[9] Shepherd, R. WO98/39003, 1998**.**
[10] Dow, G. S.; Heady, T. N.; Bhattarchargee, A. K.; Caridha, D.; Gerena, L.; Gettayacamin, M.; Lanteri, C. A.; Obaldia, N. III.; Roncal, N.; Shearer, T.; Smith, P. L.; Tungtaeng, A.; Wolf, L.; Cabezas, M.; Yourick, D.; Smith, K. S. Antimicrob. Agents Chemother. 2006, 50, 4132.
[11] Milner, E.; McCalmont, W.; Bhonsle, J.; Caridha, D.; Cobar, J.; Gardner, S.; Gerena, L.; Goodine, D.; Lanteri, C.; Melendez, V.; Roncal, N.; Sousa, J.; Wipf, P.; Dow, G. S. Malaria Journal 2010, 9, 51.
[12] (a) Milner, E.; McCalmont, W.; Bhonsle, J.; Caridha, D.; Carroll, D.; Gardner, S.; Gerena, L.; Gettayacamin, M.; Lanteri, C.; Luong, T.; Melendez, V.; Moon, J.; Ronca, N.; Sousa, J.; Tungtaeng, A.; Wipf, P.; Dow, G. S. Bioorg. Med. Chem. Lett. 2010, 20, 1347. (b) Dow, G. S.; Koenig, M. L.; Wolf, L.; Gerena, L.; Lopez-Sanchez, M.; Hudson, T. H.; Bhattacharjee, A. K. Anfimicrob. Agents Chemother. 2006, 50,4132.
[13] (a) Jayaprakash, S.; Iso, Y.; Wan, B.; Franzblau, S. G.; Kozikowski, A. P. Chem, Med. Chem. 2006, 1, 593. (b) Mao, J.; Wang, Y.; Wan, B.; Kozikowski, A. P.; Franzblau, S. G. Chem. Med. Chem. 2007, 2, 1624. Mao, J.; Yuan, H.; Wang, Y.; Wan, B.; Pak, D.; He, R.; Franzblau, S. G. Bioorg. Med. Chem. Lett. 2010, 20, 1263. (c) Kunin, C. M.; Ellis, W. Y. Antimicrob. Agents Chemother. 2000, 44, 848. (d) Vidal-Aroca, F.; Meng, A.; Minz, T., Page M. G. P.; Dreier, J. J. Microb. Methods 2009, 79, 232.
[14] (a) Gillepsie, R. J.; Lerpiniere, J.; Giles, P. R.; Adams, D. R.; Stray Knutsen, L. J.; Cliffe, I. A.; US 6,608,085 B1, Aug. 19 2003**.** (b) Gillespie, R. J.; Adams, D. R.; Bebbington, D.; Benwell, K.; Cliffe, Ian A.; Dawson, C. E.; Dourish, C. T.; Fletcher, A.; Gaur, S.; Giles, P. R.; Jordan, A. M., Knight, A. R.; Knutsen, L. J. S.; Lawrence, A.; Lerpiniere, J.; Misra, A.; Porter, R. H. P.; Pratt, R. M.; Shepherd, R.; Upton, R.; Ward, S. E.; Weissa S. M. and Williamson D. S. Bioorg. Med. Chem. Lett. 2008, 18, 2916.
[15] Lutz, R. E.; Patel, A. R.; Ohnmacht, C. J. J. Med. Chem, 1971, 14, 926.
[16] Carroll, F. I.; Blackwell, J. T. J. Med. Chem. 1974, 17, 210.
[17]Xie, Z.-X.; Zhang, L.-Z.; Ren, X.-J.; Tang, S.-Y.; Li, Y. Chin. J. Chem. 2008, 26, 1272.
[18] Alacid, E.; Nájera C. J. Org. Chem. 2008, 73, 2315-2322.
[19] Comins, D. L.; Nolan, J. M.; Bori, I. D. Tetrahedron Lett. 2005, 46, 6697.
[20] (a) Miyaura, N.; Suzuki, A. Chem. Rev. 1995, 95, 2457-2483. (b) Satoh, M.; Miyaura, N.; Suzuki, A. Chem. Lett. 1986, 8, 1329. (c) Guillon, J.; Forfar, I.; Desplat, V.; Belisle-Fabre, S.; Thiolat, D.; Massip, S.; Carrie, H.; Mossalayi, M.; Jarry, C. J. Enz. Inhib. Med. Chem. 2007, 22, 541.
[21] (a) Guillon, J.; Forfar, I.; Desplat, V.; Belisle-Fabre, S.; Thiolat, D.; Massip, S.; Carrie, H.; Mossalayi, M.; Jarry, C. J. Enz. Inhib. Med. Chem. 2007, 22, 541. (b) Molander, G. A.; Yun C. S.; Ribagorda, M.; Biolatto, B. J. Org. Chem. 2003, 68, 5534. (c) Molander, G. A.; Fumagalli, T. J. Org. Chem. 2006, 71,
5743. (d) Darses, S.; Genet, J.-P. Chem. Rev. 2008, 108, 288.
[22] For review, see: Kolb, H. C.; VanNieuwenhze, M. S.; Sharpless, K. B. Chem. Rev. 1994, 94, 2483.
[23] Kolb, H. C.; Sharpless, K. B. Tetrahedron 1992, 48, 10515.
[24] Weissman, S. A.; Rossen, K.; Reider, P. J. Org. Lett. 2001, 16, 2513-2515.
[25] Kolb, H. C.; VanNieuwenhze, M. S.; Sharpless, K. B. Chem. Rev. 1994, 94, 2483.
[26] Crystallographic data for the structure reported in this paper have been deposited with the Cambridge Crystallographic Data Centre as supplementary publication no. CCDC-780365. Copies of the data can be obtained free of charge on application to CCDC, University Chemical Lab, 12 Union Road, Cambridge, CB2 1EZ, UK (Fax: int.code + 44-1223/336-033; E-mail: deposit@ccdc.cam.ac.uk.
[27] CLSI M07-A8 Methods for dilution antimicrobial susceptibility tests for bacteria that grow aerobically; Approved standard - Eighth edition (2009)

**Claims**

**1.** A process for providing a compound of formula (I):

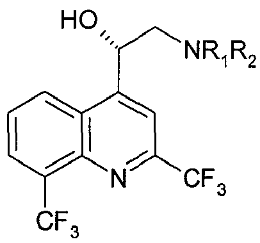

(I)

Wherein:

- $R_1$ and $R_2$ are selected from a group consisting of a hydrogen atom, a chain alkyl group ($C_1$-$C_9$), a cyclic alkyl groups and aromatic groups; or
- $NR_1R_2$ forms together a piperazine or a piperidine substituted by at least one of a hydrogen, a chain alkyl group ($C_1$-$C_9$), a cyclic alkyl group, a chain containing an aromatic group and a chain containing a heterocyclic group,

the process comprising steps of in the following order:

- preparation of 2,8-bis(trifluoromethyl)-4-vinylquinoline (3);
- preparation of (*S*)-1-[2,8-bis(trifluoromethyl)quinolin-4-yl]ethane-1,2-diol (4b) by adding an asymmetric dihydroxylation catalyst to said 2,8-bis(trifluoromethyl)-4-vinylquinoline (3);
- preparation of (*S*)-4-(oxiran-2-yl)-2,8-bis(trifluoromethyl) quinoline (2b) starting from said (*S*)-1-[2,8-bis(trifluoromethyl)quinolin-4-yl]ethane-1,2-diol (4b); and
- adding an amine $R_1R_2$ to said (*S*)-4-(oxiran-2-yl)-2,8-bis(trifluoromethyl)quinoline (2b).

**2.** A process for providing a compound of formula (II):

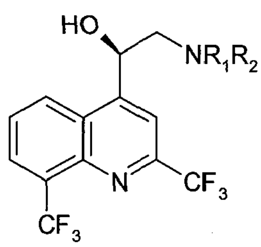

(II)

Wherein:

- $R_1$ and $R_2$ are selected from a group consisting of a hydrogen atom, a chain alkyl group ($C_1$-$C_9$), a cyclic alkyl groups and aromatic groups; or
- $NR_1R_2$ forms together a piperazine or a piperidine substituted by at least one of a hydrogen, a chain alkyl group ($C_1$-$C_9$), a cyclic alkyl group, a chain containing an aromatic group and a chain containing a heterocyclic group,

the process comprising steps of in the following order:

- preparation of 2,8-bis(trifluoromethyl)-4-vinylquinoline (3);
- preparation of (*R*)-1-[2,8-bis(trifluoromethyl)quinolin-4-yl]ethane-1,2-diol (4a) by adding an asymmetric dihydroxylation catalyst to said 2,8-bis(trifluoromethyl)-4-vinylquinoline (3);
- preparation of (*R*)-4-(oxiran-2-yl)-2,8-bis(trifluoromethyl) quinoline (2a) starting from said (*R*)-1-[2,8-bis(trifluoromethyl)quinolin-4-yl]ethane-1,2-diol (4a); and

- adding an amine $R_1R_2NH$ to said (*R*)-4-(oxiran-2-yl)-2,8-bis(trifluoromethyl)quinoline (*2*a).

**3.** The process according to claim 1, wherein the compound of formula (I) is one of (*S*)-2-(benzylamino)-1-(2,8-bis (trifluoromethyl)quinolin-4-yl)ethanol, *(S)*-ethyl-4-(4-(2-(2,8-bis(trifluoromethyl)quinolin-4-yl)-2-(hydroxyethyl)piperazin-1-yl)butanoate, (*S*)-1-(2,8-bis(trifluoromethyl)quinolin-4-yl)-2-(pentylamino)ethanol, (*S*)-1-(2,8-bis(trifluoromethyl)quinolin-4-yl)-2-(hexylamino)ethanol, (*S*)-1-(2,8-bis(trifluoromethyl)quinolin-4-yl)-2-(heptylamino)ethanol, (*S*)-1-(2,8-bis(trifluoromethyl)quinolin-4-yl)-2-(octylamino)ethanol, (*S*)-1-(2,8-bis(trifluoromethyl)quinolin-4-yl)-2-(nonylamino)ethanol, (*S*)-1-(2,8-bis(trifluoromethyl)quinolin-4-yl)-2-(piperazine substituted)ethanol or (*S*)-tert-butyl-(3-(4-(3 ( (2-(2,8- bis (trifluoromethyl) quinolin- 4- yl)- 2-(hydroxyethyl) amino) propyl) piperazin- 1- yl) propyl) carbamate.

**4.** The process according to claim 2, wherein the compound of formula (II) is one of (*R*)-2-(benzylamino)-1-(2,8-bis (trifluoromethyl)quinolin-4-yl)ethanol, (*R*)-ethyl-4-(4-(2-(2,8-bis(trifluoromethyl)quinolin-4-yl)-2-(hydroxyethyl)piperazin-1-yl)butanoate, (*R*)-1-(2,8-bis(trifluoromethyl)quinolin-4-yl)-2-(pentylamino)ethanol, (*R*)-1-(2,8-bis(trifluoromethyl)quinolin-4-yl)-2-(hexylamino)ethanol, (*R*)-1-(2,8-bis(trifluoromethyl)quinolin-4-yl)-2-(heptylamino)ethanol, (*R*)-1-(2,8-bis(trifluoromethyl)quinolin-4-yl)-2-(octylamino)ethanol, (*R*)-1-(2,8-bis(trifluoromethyl)quinolin-4-yl)-2-(nonylamino)ethanol (*R*)-1-(2,8-bis(trifluoromethyl)quinolin-4-yl)-2-(piperazine substituted) ethanol or (*R*)-tert-butyl-(3-(4-(3 ( (2-(2,8- bis (trifluoromethyl) quinolin- 4- yl)- 2-(hydroxyethyl) amino) propyl) piperazin- 1- yl) propyl) carbamate.

**5.** The process according to claim 1 or 2 wherein said 2,8-bis(trifluoromethyl)-4-vinylquinoline (3) is prepared by reacting a 4-bromo-2,8-bis(trifluoromethyl)quinoline (6a) with vinylstannane using the $Pd(PPh_3)_2Cl_2$ as - catalyst and tetrabutylammonium bromide as additive in reflux of dried acetonitrile.

**6.** The process according to claim 1 or 2 wherein said 2,8-bis(trifluoromethyl)-4-vinylquinoline (3) is prepared by reacting 4-bromo-2,8-bis(trifluoromethyl)quinoline (6a) with dibutyl vinylboronate.

**7.** The process according to claim 1 or 2 wherein said 2,8-bis(trifluoromethyl)-4-vinylquinoline (3) is prepared by reacting 4-bromo-2,8-bis(trifluoromethyl)quinoline (6a) with potassium vinyltrifluoroborate by using $PdCl_2$(dppf) $CH_2Cl_2$ as the catalyst, $Cs_2CO_3$ as the base, and THF-$H_2O$ as the solvent system.

**8.** The process according to any one of claims 1 to 7, wherein:

said asymmetric dihydroxylation catalysts is one of AD-mix $\alpha$ and AD-mix $\beta$;
said (*S*) -1-[2,8-bis(trifluoromethyl)quinolin-4-yl]ethane-1,2-diol (4b) is prepared by reacting said 2,8-bis(trifluoromethyl)-4-vinylquinoline (3) with AD-mix $\alpha$ in a solution of t-BuOH/$H_2O$ at 0˚C; and
said (*R*) -1-[2,8-bis(trifluoromethyl)qinolin-4-yl]ethane-1,2-diol (4a) is prepared by reacting said 2,8-bis(trifluoromethyl)-4-vinylquinoline (3) with AD-mix $\beta$ in a solution of t-BuOH/$H_2O$ at 0˚C.

**9.** The process according to claim 8 wherein $K_2OsO_2(OH)_4$ is further added to prepare said (*S*)-1-[2,8-bis(trifluoromethyl)quinolin-4-yl]ethane-1,2-diol (4b) and said (*R*) -1-[2,8-bis(trifluoromethyl)quinolin-4-yl]ethane-1,2-diol (4a).

**10.** The process according to any one of claims 1 to 9, wherein starting from respectively (*S*)-1-[2,8-bis(trifluoromethyl) quinolin-4-yl]ethane-1,2-diol (4b) and *(R)*-1-[2,8-bis(trifluoromethyl)quinolin-4-yl]ethane-1,2-diol (4a), said respectively *(S)*-4-(oxiran-2-yl)-2,8-bis(trifluoromethyl) quinoline (2b) and *(R)*-4-(oxiran-2-yl)-2,8-bis(trifluoromethyl) quinoline (2a) are prepared by a process comprising steps of:

- formation of the cyclic orthoester by acid catalyzed transesterification;
- generation of halohydrins ester via regioselective opening of acetoxonium ion by addition of tributyldimethylsilyl chloride; and
- cyclization to epoxide by base mediated saponification in methanol.

**11.** The compound obtainable by the process according to claims 1 to 10.

**12.** The enantiopure compound of formula (I) or (II):

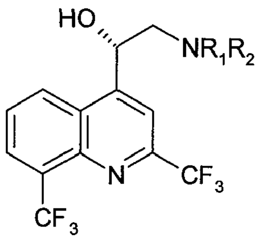

(I)

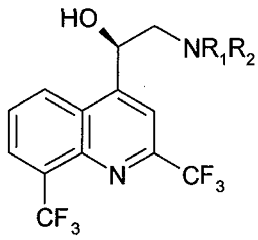

(II)

Wherein:

- $R_1$ and $R_2$ are selected from a group consisting of a hydrogen atom, a chain alkyl group ($C_1$-$C_9$), a cyclic alkyl groups and aromatic groups; or
- $NR_1R_2$ forms together a piperazine or a piperidine substituted by at least one of a hydrogen, a chain alkyl group ($C_1$-$C_9$), a cyclic alkyl group, a chain containing an aromatic group and a chain containing a heterocyclic group.

**13.** The compound according to claim 12, wherein the compound of formula (I) is one of *(S)*-2-(benzylamino)-1-(2,8-bis(trifluoromethyl)quinolin-4-yl)ethanol, *(S)*-ethyl-4-(4-(2-(2,8-bis(trifluoromethyl)quinolin-4-yl)-2-(hydroxyethyl) piperazin-1-yl)butanoate, *(S)*-1-(2,8-bis(trifluoromethyl)quinolin-4-yl)-2-(pentylamino)ethanol, *(S)*-1-(2,8-bis(trifluoromethyl)quinolin-4-yl)-2-(hexylamino)ethanol, *(S)*-1-(2,8-bis(trifluoromethyl)quinolin-4-yl)-2-(heptylamino)ethanol, *(S)*-1-(2,8-bis(trifluoromethyl)quinolin-4-yl)-2-(octylamino)ethanol, *(S)*-1-(2,8-bis(trifluoromethyl)quinolin-4-yl)-2-(nonylamino)ethanol, *(S)*-1-(2,8-bis(trifluoromethyl)quinolin-4-yl)-2-(piperazine substituted)ethanol or *(S)*-tert-butyl-(3-(4-(3 ( (2-(2,8- bis (trifluoromethyl) quinolin- 4- yl)- 2-(hydroxyethyl) amino) propyl) piperazin- 1- yl) propyl) carbamate..

**14.** The compound according to claim 12, wherein the compound of formula (II) is one of *(R)*-2-(benzylamino)-1-(2,8-bis(trifluoromethyl)quinolin-4-yl)ethanol, *(R)*-ethyl-4-(4-(2-(2,8-bis(trifluoromethyl)quinolin-4-yl)-2-(hydroxyethyl) piperazin-1-yl)butanoate, *(R)*-1-(2,8-bis(trifluoromethyl)quinolin-4-yl)-2-(pentylamino)ethanol, *(R)*-1-(2,8-bis(trifluoromethyl)quinolin-4-yl)-2-(hexylamino)ethanol, *(R)*-1-(2,8-bis(trifluoromethyl)quinolin-4-yl)-2-(heptylamino)ethanol, *(R)*-1-(2,8-bis(trifluoromethyl)quinolin-4-yl)-2-(octylamino)ethanol, *(R)*-1-(2,8-bis(trifluoromethyl)quinolin-4-yl)-2-(nonylamino)ethanol, *(R)*-1-(2,8-bis(trifluoromethyl)quinolin-4-yl)-2-(piperazine substituted) ethanol or *(R)*-tert-butyl-(3-(4-(3 ( (2-(2,8- bis (trifluoromethyl) quinolin- 4- yl)- 2-(hydroxyethyl) amino) propyl) piperazin- 1- yl) propyl) carbamate.

**15.** The compound according to any one of claims 11 to 14, for the use as an antimalarial agent.

**16.** The compound according to any one of claims 11 to 14, for the use as an antibacterial agent.

**17.** A pharmaceutical composition comprising the compound according to any one of claims 11 to 14, and/or pharmaceutically acceptable salts thereof.

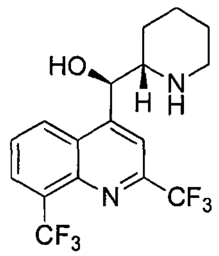

(+)-enantiomer
(+)-(11*R*, 12*S*)

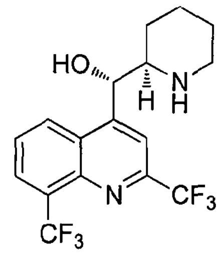

(-)-enantiomer
(-)-(11*S*, 12*R*)

**Figure 1.** Mefloquine Enantiomer Structures.

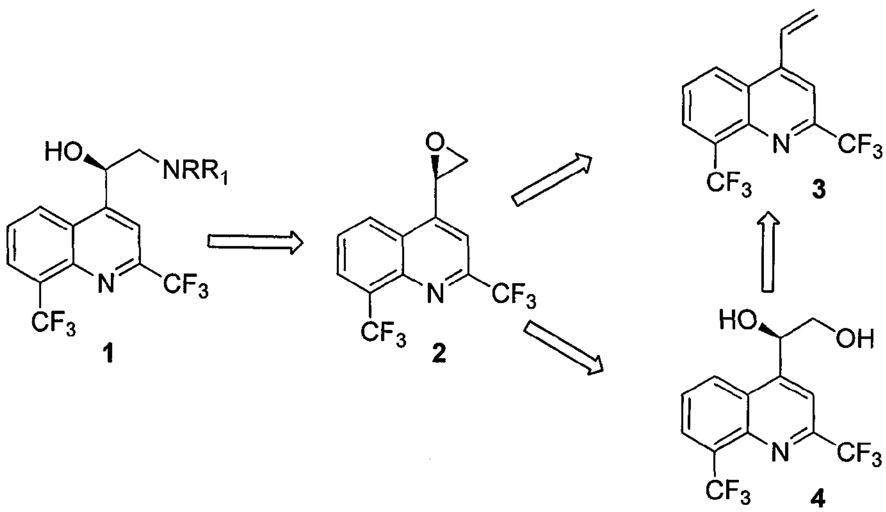

1 2 3 4

**Figure 2 .** Retrosynthesis of (11*R*)- Enantiopure 4-Aminoalcoholquinoline **1.**

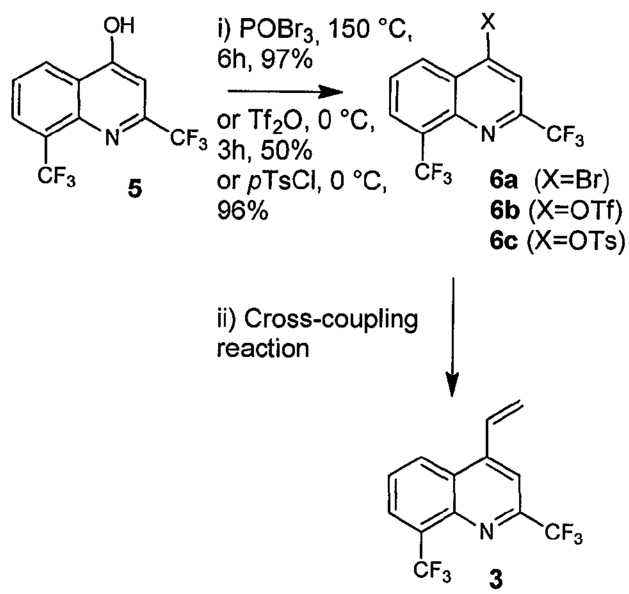


5

**6a** (X=Br)
**6b** (X=OTf)
**6c** (X=OTs)

3

**Figure 3.** Synthesis of 2,8-trifluoromethyl-4-vinylquinoline **3.**

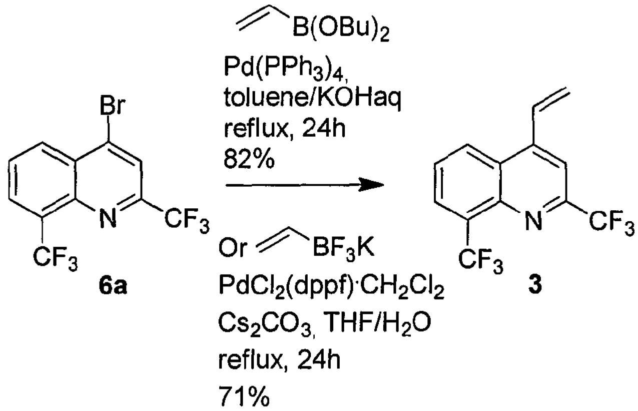


**Figure 4. Suzuki Cross-Coupling.**

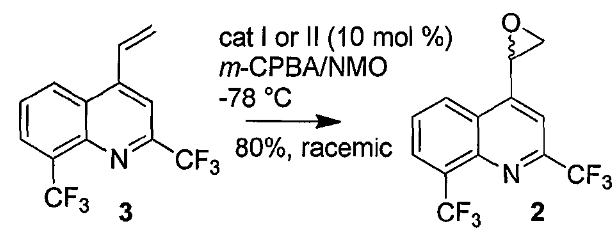


Cat I

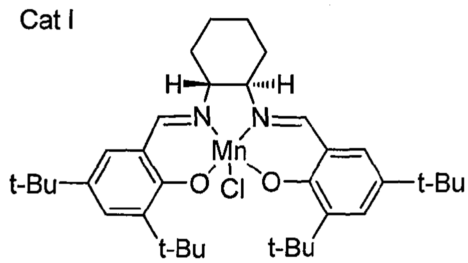

Cat II

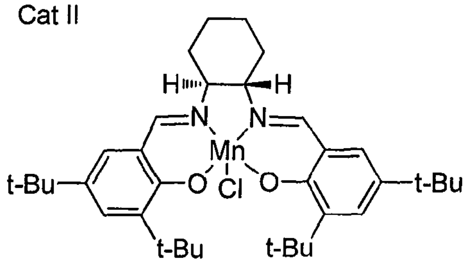

**Figure 5. Direct Oxirane Synthesis Using the Chiral (salen)Mn(III) Complexes.**

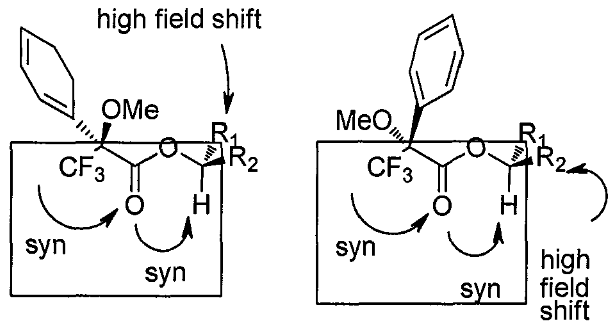


(*S*,*X*)-ester mosher (*R*,*X*)-ester mosher

**Figure 6.** Preferred Conformations of MTPA Esters

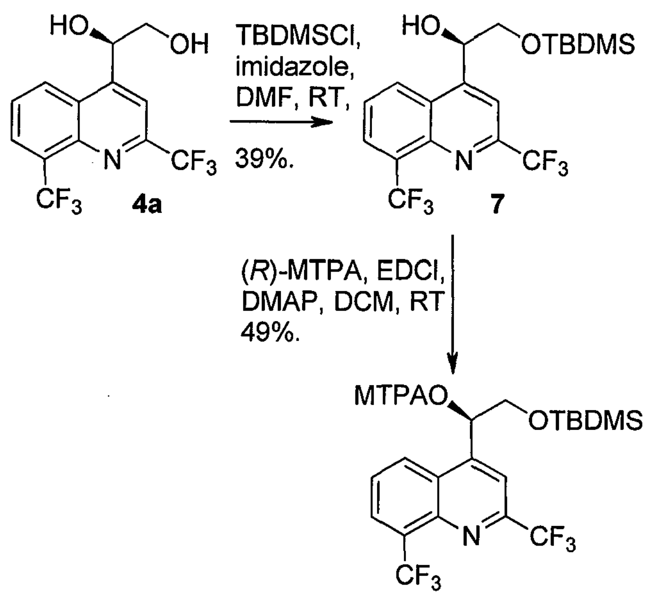


**Figure 7.** Preparation of Mosher's monoester **8a** and **8b.**

$\Delta\delta = \delta(S,X) - \delta(R,X)$

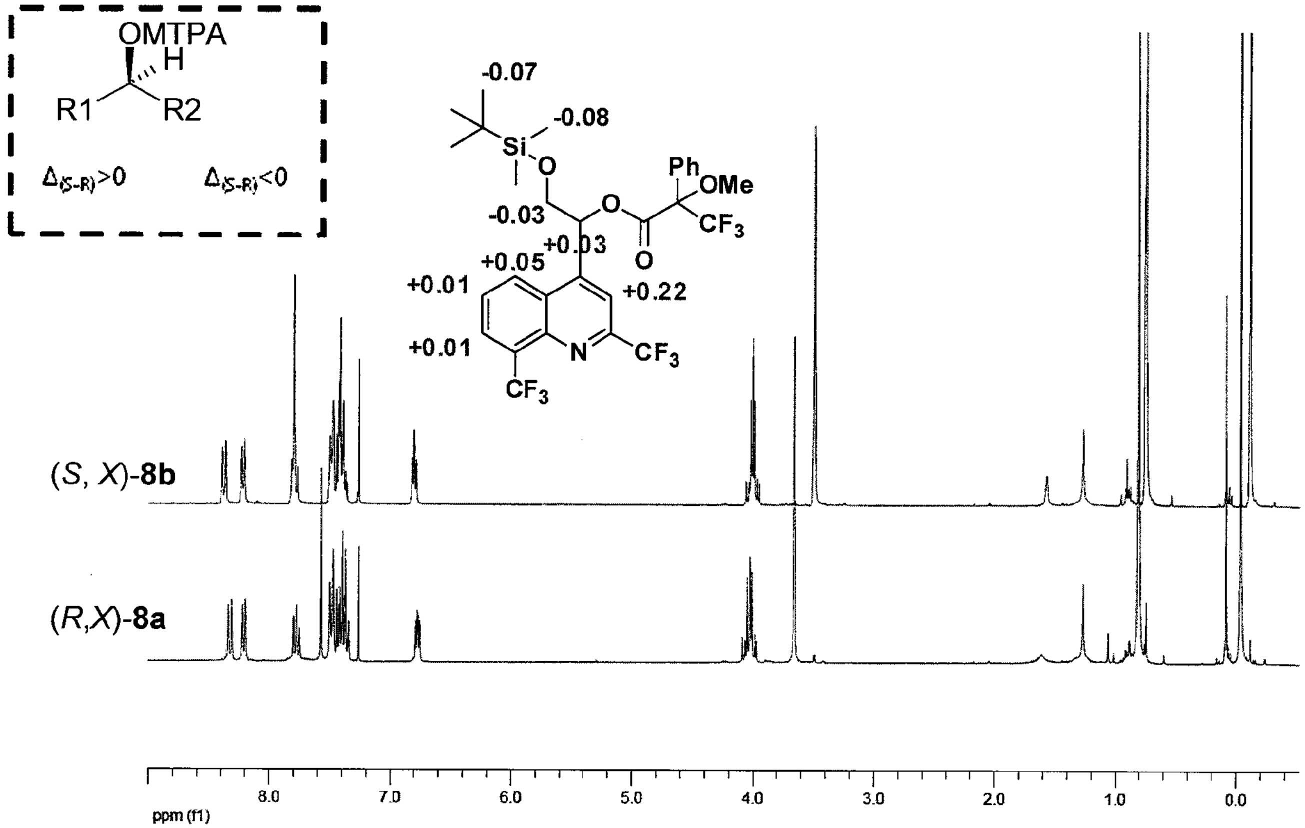


**Figure 8.** $^{1}$H NMR Analysis of the Diastereomeric Esters **8a** and **8b**.

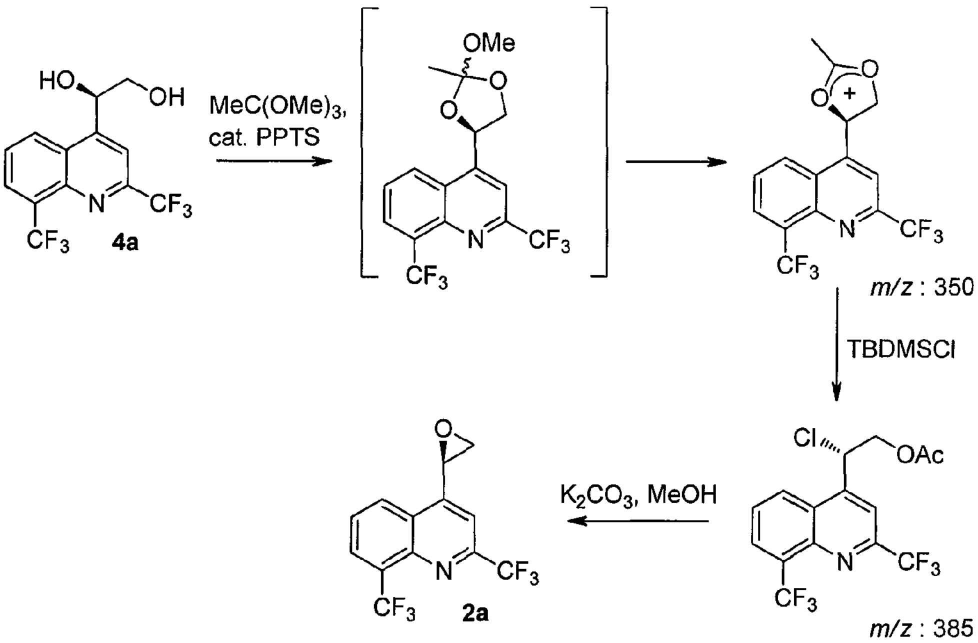


4a

2a

**Figure. 9** One-pot Stereospecific Conversion of the 1,2-diol **4a** into Epoxyde **2a** *via* a Chlorhydrine ester.

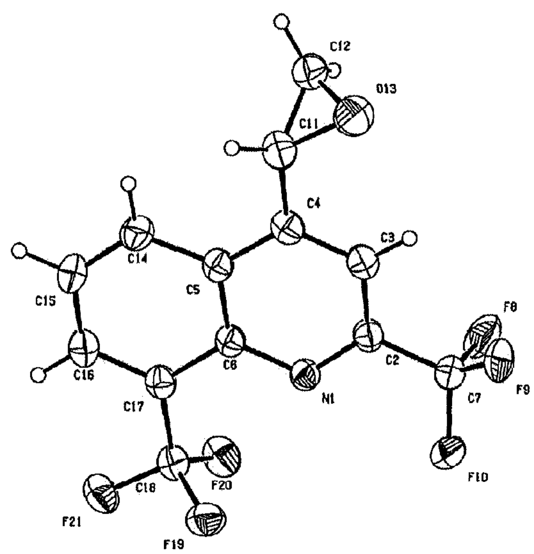

**Figure 10.** View of the Crystal Structure of **(*R*)-2a** with our Numbering Scheme, Displacement Ellipsoids Are Drawn at the 30% Probability Level.

Europäisches Patentamt
European Patent Office
Office européen des brevets

# EUROPEAN SEARCH REPORT

Application Number
EP 11 15 4229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MILNER E ET AL: "Structureactivity relationships amongst 4-position quinoline methanol antimalarials that inhibit the growth of drug sensitive and resistant strains of Plasmodium falciparum", BIOORGANIC & MEDICINAL CHEMISTRY LETTERS, PERGAMON, ELSEVIER SCIENCE, GB, vol. 20, no. 4, 15 February 2010 (2010-02-15), pages 1347-1351, XP026887067, ISSN: 0960-894X, DOI: DOI:10.1016/J.BMCL.2010.01.001 [retrieved on 2010-01-07] | 11-17 | INV. C07D215/14 A61K31/47 A61P33/06 A61P31/04 |
| Y | * Whole document; in particular Tables 1-6 and Figure 1 and pages 1347-1348 * | 1-10 | |
| | ----- | | |
| X | WO 2010/144101 A1 (US OF AMERICA AS REPRESENTED BY THE SECRETARY OF THE ARMY ON BEHALF OF) 16 December 2010 (2010-12-16) | 11-17 | |
| A | * Claims 1, 16-18; Tables 1-3; Figure 2 and page 16 paragraph [0082] * | 1-10 | TECHNICAL FIELDS SEARCHED (IPC) |
| | ----- | | C07D |
| X,D | US 6 608 085 B1 (GILLESPIE ROGER JOHN [GB] ET AL) 19 August 2003 (2003-08-19) | 11-17 | |
| A | * Claims and examples 19, 22-26 * | 1-10 | |
| | ----- | | |
| X | WO 2008/060269 A2 (US ARMY [US]; DOW GEOFFREY S [US]; SMITH KIRSTEN [US]; HEADY TIFFANY N) 22 May 2008 (2008-05-22) | 11,12, 15-17 | |
| A | * claims 1-6, 9, 29; compounds (III)(a), (V)(c) * | 1-10,13, 14 | |
| | ----- | | |
| | -/-- | | |

The present search report has been drawn up for all claims

2

| Place of search | Date of completion of the search | Examiner |
|---|---|---|
| Munich | 28 April 2011 | Lécaillon, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 15 4229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BLUMBERGS P ET AL: "ANTIMALARIALS. 7. 2,8-BIS(TRIFLUOROMETHYL)-4-QUINOLINEMETHANOLS", JOURNAL OF MEDICINAL CHEMISTRY, AMERICAN CHEMICAL SOCIETY, WASHINGTON, US, vol. 18, no. 11, 1 January 1975 (1975-01-01), pages 1122-1126, XP000915125, ISSN: 0022-2623, DOI: DOI:10.1021/JM00245A015 | 11,12, 15-17 | |
| A | * Whole document and in particular Table I compounds (3), (5)-(9) * | 1-10,13, 14 | |
| X,D | MAO, J.WANG, Y.WAN, B.KOZIKOWSKI, A. P.FRANZBLAU, S. G.: "Design, Symthesis, and Pharmacological Evaluation of Mefloquine-Based Ligands as Novel Antituberculosis Agents", CHEM. MED. CHEM., vol. 2, 2007, pages 1624-1630, XP002634483, | 11-17 | |
| A | * compound (9a) * | 1-10 | TECHNICAL FIELDS SEARCHED (IPC) |
| X | WO 2010/144434 A1 (US OF AMERICA AS REPRESENTED BY THE SECRETARY OF THE ARMY ON BEHALF OF) 16 December 2010 (2010-12-16) | 11-17 | |
| Y | * Description pages 6-7 and figures * * Figure 33 * | 1-10 | |

-/--

The present search report has been drawn up for all claims

| Place of search | Date of completion of the search | Examiner |
|---|---|---|
| Munich | 28 April 2011 | Lécaillon, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 15 4229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | KOLB H C ET AL: "A Simplified Procedure for the Stereospecific Transformation of 1,2-Diols into Epoxides", TETRAHEDRON, ELSEVIER SCIENCE PUBLISHERS, AMSTERDAM, NL, vol. 48, no. 48, 1 January 1992 (1992-01-01), pages 10515-10530, XP002993847, ISSN: 0040-4020, DOI: DOI:10.1016/S0040-4020(01)88349-6 * the whole document * ----- | 10 | |
| Y,D | KOLB H C ET AL: "CATALYTIC ASYMMETRIC DIHYDROXYLATION", CHEMICAL REVIEWS, ACS,WASHINGTON, DC, US, vol. 94, 1 January 1994 (1994-01-01), pages 2483-2547, XP000857717, ISSN: 0009-2665, DOI: DOI:10.1021/CR00032A009 * the whole document * ----- | 1-10 | |
| Y,D | DARSES J. AND GENET J-P.: "Potassium Organotrifluoroborates: New Perspectives in Organic Synthesis", CHEMICAL REVIEWS, vol. 108, 2008, pages 288-325, XP002634484, * Pages 307-308 and Scheme 86 * ----- -/-- | 7 | TECHNICAL FIELDS SEARCHED (IPC) |

The present search report has been drawn up for all claims

| Place of search | Date of completion of the search | Examiner |
|---|---|---|
| Munich | 28 April 2011 | Lécaillon, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 15 4229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | MIYAURA N ET AL: "PALLADIUM-CATALYZED CROSS-COUPLING REACTIONS OF ORGANOBORON COMPOUNDS", CHEMICAL REVIEWS, ACS,WASHINGTON, DC, US, vol. 95, no. 7, 1 January 1995 (1995-01-01), pages 2457-2483, XP000652239, ISSN: 0009-2665, DOI: DOI:10.1021/CR00039A007 * Page 2469 Equation (67) * ----- | 6 | |
| Y,D | COMINS D L ET AL: "Regioselective lithium-halogen exchange and palladium-catalyzed cross-coupling reactions of 2,4-dihaloquinolines", TETRAHEDRON LETTERS, ELSEVIER, AMSTERDAM, NL, vol. 46, no. 39, 26 September 2005 (2005-09-26), pages 6697-6699, XP025386480, ISSN: 0040-4039, DOI: DOI:10.1016/J.TETLET.2005.07.137 [retrieved on 2005-09-26] * Page 6698 Table 2 Entry 2 * ----- | 5 | |

TECHNICAL FIELDS SEARCHED (IPC)

The present search report has been drawn up for all claims

| Place of search | Date of completion of the search | Examiner |
|---|---|---|
| Munich | 28 April 2011 | Lécaillon, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 11 15 4229

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2011

| Patent document cited in search report | Publication date | Patent family member(s) | Publication date |
|---|---|---|---|
| WO 2010144101 A1 | 16-12-2010 | NONE | |
| US 6608085 B1 | 19-08-2003 | AU 5640299 A | 27-03-2000 |
| | | EP 1107761 A2 | 20-06-2001 |
| | | WO 0013682 A2 | 16-03-2000 |
| WO 2008060269 A2 | 22-05-2008 | US 2011092488 A1 | 21-04-2011 |
| WO 2010144434 A1 | 16-12-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9839003 A, Shepherd, R.
- US 6608085 B1, Gillepsie, R. J.; Lerpiniere, J.; Giles, P. R.; Adams, D. R.; Stray Knutsen, L. J.; Cliffe, I. A.

### Non-patent literature cited in the description


- **BHATTACHARJEE, A. K. ; KARLE, J. M.** *J. Med. Chem.,* 2002, vol. 39, 4622
- **GO, M. L.** *Med. Res. Rev.,* 2003, vol. 23, 456
- **BIOT, C. ; CHIBALE, K.** *Infect. Disorders-Drug Targets,* 2006, vol. 6, 173
- **KARLE, J. M. ; OLMEDA, R. ; GERENA, L. ; MILHOUS, W. K.** *Exp. Pharmacol.,* 1993, vol. 76, 345
- **PHILLIPS-HOWARD, P. A. ; TER KUILE, F. O.** *Drug Saf.,* 1995, 370
- **WEINKE, T. ; TRAUTMANN, M. ; HELD, T. ; WEBER, G. ; EICHENLAUB, D. ; FLEISCHER, K. ; KERN, W. ; POLHE, H. D.** *Am. J. Trop. Med. Hyg.,* 1991, vol. 45, 86
- **VAN RIEMSDIJK, M. M. ; VAN DER KLAUW, M. M. ; VAN HEEST, J. A. ; REEDEKER, F. R. ; LIGTHELM, R. J. ; HERINGS, R. M. ; STRICKER, B. H.** *Eur. J. Clin. Pharmacol.,* 1997, vol. 52, 1
- **DOW, G. S. ; HEADY, T. N. ; BHATTARCHARGEE, A. K. ; CARIDHA, D. ; GERENA, L. ; GETTAYACAMIN, M. ; LANTERI, C. A. ; OBALDIA, N. III. ; RONCAL, N. ; SHEARER, T.** *Antimicrob. Agents Chemother.,* 2006, vol. 50, 4132
- **MILNER, E. ; MCCALMONT, W. ; BHONSLE, J. ; CARIDHA, D. ; COBAR, J. ; GARDNER, S. ; GERENA, L. ; GOODINE, D. ; LANTERI, C. ; MELENDEZ, V.** *Malaria Journal,* 2010, vol. 9, 51
- **MILNER, E. ; MCCALMONT, W. ; BHONSLE, J. ; CARIDHA, D. ; CARROLL, D. ; GARDNER, S. ; GERENA, L. ; GETTAYACAMIN, M. ; LANTERI, C. ; LUONG, T.** *Bioorg. Med. Chem. Lett.,* 2010, vol. 20, 1347
- **DOW, G. S. ; KOENIG, M. L. ; WOLF, L. ; GERENA, L. ; LOPEZ-SANCHEZ, M. ; HUDSON, T. H. ; BHATTACHARJEE, A. K.** *Anfimicrob. Agents Chemother.,* 2006, vol. 50, 4132
- **JAYAPRAKASH, S. ; ISO, Y. ; WAN, B. ; FRANZBLAU, S. G. ; KOZIKOWSKI, A. P.** *Chem, Med. Chem.,* 2006, vol. 1, 593
- **MAO, J. ; WANG, Y. ; WAN, B. ; KOZIKOWSKI, A. P. ; FRANZBLAU, S. G.** *Chem. Med. Chem.,* 2007, vol. 2, 1624
- **MAO, J. ; YUAN, H. ; WANG, Y. ; WAN, B. ; PAK, D. ; HE, R. ; FRANZBLAU, S. G.** *Bioorg. Med. Chem. Lett.,* 2010, vol. 20, 1263
- **KUNIN, C. M. ; ELLIS, W. Y.** *Antimicrob. Agents Chemother.,* 2000, vol. 44, 848
- **VIDAL-AROCA, F. ; MENG, A. ; MINZ, T. ; PAGE M. G. P. ; DREIER, J.** *J. Microb. Methods,* 2009, vol. 79, 232
- **GILLESPIE, R. J. ; ADAMS, D. R. ; BEBBINGTON, D. ; BENWELL, K. ; CLIFFE, IAN A. ; DAWSON, C. E. ; DOURISH, C. T. ; FLETCHER, A. ; GAUR, S. ; GILES, P. R.** *Bioorg. Med. Chem. Lett.,* 2008, vol. 18, 2916
- **LUTZ, R. E. ; PATEL, A. R. ; OHNMACHT, C. J.** *J. Med. Chem,* 1971, vol. 14, 926
- **CARROLL, F. I. ; BLACKWELL, J. T.** *J. Med. Chem.,* 1974, vol. 17, 210
- **XIE, Z.-X. ; ZHANG, L.-Z. ; REN, X.-J. ; TANG, S.-Y. ; LI, Y.** *Chin. J. Chem.,* 2008, vol. 26, 1272
- **ALACID, E. ; NÁJERA C.** *J. Org. Chem.,* 2008, vol. 73, 2315-2322
- **COMINS, D. L. ; NOLAN, J. M. ; BORI, I. D.** *Tetrahedron Lett.,* 2005, vol. 46, 6697
- **MIYAURA, N. ; SUZUKI, A.** *Chem. Rev.,* 1995, vol. 95, 2457-2483
- **SATOH, M. ; MIYAURA, N. ; SUZUKI, A.** *Chem. Lett.,* 1986, vol. 8, 1329
- **GUILLON, J. ; FORFAR, I. ; DESPLAT, V. ; BELISLE-FABRE, S. ; THIOLAT, D. ; MASSIP, S. ; CARRIE, H. ; MOSSALAYI, M. ; JARRY, C.** *J. Enz. Inhib. Med. Chem.,* 2007, vol. 22, 541
- **MOLANDER, G. A. ; YUN C. S. ; RIBAGORDA, M. ; BIOLATTO, B.** *J. Org. Chem.,* 2003, vol. 68, 5534
- **MOLANDER, G. A. ; FUMAGALLI, T.** *J. Org. Chem.,* 2006, vol. 71
- **DARSES, S. ; GENET, J.-P.** *Chem. Rev.,* 2008, vol. 108, 288
- **KOLB, H. C. ; VANNIEUWENHZE, M. S. ; SHARPLESS, K. B.** *Chem. Rev.,* 1994, vol. 94, 2483

- **KOLB, H. C. ; SHARPLESS, K. B.** *Tetrahedron,* 1992, vol. 48, 10515
- **WEISSMAN, S. A. ; ROSSEN, K. ; REIDER, P.** *J. Org. Lett.,* 2001, vol. 16, 2513-2515